# EUROPEAN PATENT SPECIFICATION

(11) **EP 1 747 276 B1**
(45) Date of publication and mention of the grant of the patent: **10.12.2008**
(21) Application number: 05746397.8
(22) Date of filing: 06.05.2005
(51) Int. Cl.: C12N 15/82, C12N 9/02, A01H 5/00

(54) **CAROTENOID BIOSYNTHESIS INHIBITOR RESISTANCE GENES AND METHODS OF USE IN PLANTS**
CAROTINOID-BIOSYNTHESENHEMMUNGS-RESISTENZGEN UND VERFAHREN ZUR VERWENDUNG IN PFLANZEN
GENES DE RESISTANCE AUX INHIBITEURS DE LA BIOSYNTHESE DES CAROTENOIDES ET LEURS METHODES D'UTILISATION DANS DES PLANTES

(30) Priority: 07.05.2004 US 569489 P
(43) Date of publication of application: 31.01.2007
(73) Proprietor: BASF Plant Science GmbH, 67056 Ludwigshafen (DE)
(72) Inventor: ZHEN, Rui-Guang, Chapel Hill, NC 27516 (US); HIRAYAMA, Lynne, Titusville, NJ 08560 (US); PENG, Jianying, Durham, NC 27713 (US); POGREBNYAK, Natalia, Highland Park, NJ 08904 (US); CARPENTER, Brian, Morrisville, PA 19067 (US); COZZITORTO, Joseph, A., Jr., Monmouth Junction, NJ 08852 (US); BROWN, Kevin, R., Lansdale, PA 19446 (US); HEDRICK, Jean, South River, NJ 08882 (US); STAHL, Geraldine, Huntsville, AR 72740 (US)
(74) Representative: Popp, Andreas
(86) International application number: PCT/US2005/015767
(87) International publication number: WO 2005/111218

(56) References cited:
- EP-A- 1 033 405
- WO-A-02/00915
- WO-A-99/53081
- WO-A-20/04007691
- CHAMOVITZ D ET AL: "THE MOLECULAR BASIS OF RESISTANCE TO THE HERBICIDE NORFLURAZON" PLANT MOLECULAR BIOLOGY, SPRINGER, DORDRECHT, NL, vol. 16, 1991, pages 967-974, XP000881035 ISSN: 0167-4412
- WAGNER TOBIAS ET AL: "Transformation of tobacco with a mutated cyanobacterial phytoene desaturase gene confers resistance to bleaching herbicides" ZEITSCHRIFT FUER NATURFORSCHUNG SECTION C JOURNAL OF BIOSCIENCES, vol. 57, no. 7-8, July 2002 (2002-07), pages 671-679, XP002350957 ISSN: 0939-5075

### SEQUENCE LISTING

<110> BASF PLANT SCIENCE GMBH
<120> CAROTENOID BIOSYNTHESIS INHIBITOR RESISTANCE GENES AND METHODS OF USE IN PLANTS
<130> 15044
<140>
   <141>
<150> 60/569,489
   <151> 2004-05-07
<160> 8
<170> PatentIn Ver. 3.3
<210> 1
   <211> 1698
   <212> DNA
   <213> Arabidopsis thaliana
<400> 1
   atggttgtgt ttgggaatgt ttctgcggcg aatttgcctt atcaaaacgg gtttttggag 60
   gcactttcat ctggaggttg tgaactaatg ggacatagct ttagggttcc cacttctcaa 120
   gcgcttaaga caagaacaag gaggaggagt actgctggtc ctttgcaggt agtttgtgtg 180
   gatattccaa ggccagagct agagaacact gtcaatttct tggaagctgc tagtttatct 240
   gcatccttcc gtagtgctcc tcgtcctgct aagcctttga aagttgtaat tgctggtgct 300
   ggattggctg gattgtcaac tgcaaagtac ctggctgatg caggccacaa acctctgttg 360
   cttgaagcaa gagatgttct tggtggaaag atagctgcat ggaaggatga agatggggac 420
   tggtatgaga ctggtttaca tattttcttc ggtgcttatc cgaatgtgca gaatttattt 480
   ggagaacttg ggatcaatga tcggttgcag tggaaggaac actccatgat ttttgctatg 540
   ccaagtaaac ctggagaatt tagtagattt gacttcccag atgtcctacc agcaccctta 600
   aatggtattt gggctatttt gcggaacaac gagatgctga catggccaga gaaaataaag 660
   tttgctattg gacttttgcc agccatggtc ggcggtcagg cttatgttga ggcccaagat 720
   ggtttatcag tcaaagaatg gatggaaaag cagggagtac ctgagcgcgt gaccgacgag 780
   gtgtttattg ccatgtcaaa ggcgctaaac tttataaacc ctgatgaact gtcaatgcaa 840
   tgcattttga tagctttgaa cccctttctt caggaaaaac atggttccaa gatggcattc 900
   ttggatggta atcctccgga aaggctttgt atgccagtag tggatcatat tcgatcacta 960
   ggtggggaag tgcaacttaa ttctaggata aagaaaattg agctcaatga cgatggcacg 1020
   gttaagagtt tcttactcac taatggaagc actgtcgaag gagacgctta tgtgtttgcc 1080
   gctccagtcg atatcctgaa gctcctttta ccagatccct ggaaagaaat accgtacttc 1140
   aagaaattgg ataaattagt tggagtacca gttattaatg ttcatatatg gtttgatcga 1200
   aaactgaaga acacatatga tcacctactc tttagcagaa gtaaccttct gagcgtgtat 1260
   gccgacatgt ccttaacttg taaggaatat tacgatccta accggtcaat gctggagcta 1320
   gtatttgcac cagcagagga atggatatca cggactgatt ctgacatcat agatgcaaca 1380
   atgaaagaac ttgagaaact cttccctgat gaaatctcag ctgaccaaag caaagctaaa 1440
   attctgaagt accatgtcgt taagactcca agatctgtgt acaagaccat cccaaactgt 1500
   gaaccatgtc gtcctctaca aagatcacct attgaaggat tctacttagc tggagattac 1560
   acaaaacaga agtacttagc ttccatggaa ggcgctgtcc tctctggcaa attctgctct 1620
   cagtctattg ttcaggatta cgagctactg gctgcgtctg gaccaagaaa gttgtcggag 1680
   gcaacagtat catcatca                                               1698
<210> 2
   <211> 566
   <212> PRT
   <213> Arabidopsis thaliana
<400> 2
   Met Val Val Phe Gly Asn Val Ser Ala Ala Asn Leu Pro Tyr Gln Asn
   1               5                   10                  15
   Gly Phe Leu Glu Ala Leu Ser Ser Gly Gly Cys Glu Leu Met Gly His
               20                  25                  30
   Ser Phe Arg Val Pro Thr Ser Gln Ala Leu Lys Thr Arg Thr Arg Arg
           35                  40                  45
   Arg Ser Thr Ala Gly Pro Leu Gln Val Val Cys Val Asp Ile Pro Arg
       50                  55                  60
   Pro Glu Leu Glu Asn Thr Val Asn Phe Leu Glu Ala Ala Ser Leu Ser
   65                  70                  75                  80
   Ala Ser Phe Arg Ser Ala Pro Arg Pro Ala Lys Pro Leu Lys Val Val
                   85                  90                  95
   Ile Ala Gly Ala Gly Leu Ala Gly Leu Ser Thr Ala Lys Tyr Leu Ala
               100                 105                 110
   Asp Ala Gly His Lys Pro Leu Leu Leu Glu Ala Arg Asp Val Leu Gly
           115                 120                 125
   Gly Lys Ile Ala Ala Trp Lys Asp Glu Asp Gly Asp Trp Tyr Glu Thr
       130                 135                 140
   Gly Leu His Ile Phe Phe Gly Ala Tyr Pro Asn Val Gln Asn Leu Phe
   145                 150                 155                 160
   Gly Glu Leu Gly Ile Asn Asp Arg Leu Gln Trp Lys Glu His Ser Met
                   165                 170                 175
   Ile Phe Ala Met Pro Ser Lys Pro Gly Glu Phe Ser Arg Phe Asp Phe
               180                 185                 190
   Pro Asp Val Leu Pro Ala Pro Leu Asn Gly Ile Trp Ala Ile Leu Arg
           195                 200                 205
   Asn Asn Glu Met Leu Thr Trp Pro Glu Lys Ile Lys Phe Ala Ile Gly
       210                 215                 220
   Leu Leu Pro Ala Met Val Gly Gly Gln Ala Tyr Val Glu Ala Gln Asp
   225                 230                 235                 240
   Gly Leu Ser Val Lys Glu Trp Met Glu Lys Gln Gly Val Pro Glu Arg
                   245                 250                 255
   Val Thr Asp Glu Val Phe Ile Ala Met Ser Lys Ala Leu Asn Phe Ile
               260                 265                 270
   Asn Pro Asp Glu Leu Ser Met Gln Cys Ile Leu Ile Ala Leu Asn Pro
           275                 280                 285
   Phe Leu Gln Glu Lys His Gly Ser Lys Met Ala Phe Leu Asp Gly Asn
       290                 295                 300
   Pro Pro Glu Arg Leu Cys Met Pro Val Val Asp His Ile Arg Ser Leu
   305                 310                 315                 320
   Gly Gly Glu Val Gln Leu Asn Ser Arg Ile Lys Lys Ile Glu Leu Asn
                   325                 330                 335
   Asp Asp Gly Thr Val Lys Ser Phe Leu Leu Thr Asn Gly Ser Thr Val
               340                 345                 350
   Glu Gly Asp Ala Tyr Val Phe Ala Ala Pro Val Asp Ile Leu Lys Leu
           355                 360                 365
   Leu Leu Pro Asp Pro Trp Lys Glu Ile Pro Tyr Phe Lys Lys Leu Asp
       370                 375                 380
   Lys Leu Val Gly Val Pro Val Ile Asn Val His Ile Trp Phe Asp Arg
   385                 390                 395                 400
   Lys Leu Lys Asn Thr Tyr Asp His Leu Leu Phe Ser Arg Ser Asn Leu
                   405                 410                 415
   Leu Ser Val Tyr Ala Asp Met Ser Leu Thr Cys Lys Glu Tyr Tyr Asp
               420                 425                 430
   Pro Asn Arg Ser Met Leu Glu Leu Val Phe Ala Pro Ala Glu Glu Trp
           435                 440                 445
   Ile Ser Arg Thr Asp Ser Asp Ile Ile Asp Ala Thr Met Lys Glu Leu
       450                 455                 460
   Glu Lys Leu Phe Pro Asp Glu Ile Ser Ala Asp Gln Ser Lys Ala Lys
   465                 470                 475                 480
   Ile Leu Lys Tyr His Val Val Lys Thr Pro Arg Ser Val Tyr Lys Thr
                   485                 490                 495
   Ile Pro Asn Cys Glu Pro Cys Arg Pro Leu Gln Arg Ser Pro Ile Glu
               500                 505                 510
   Gly Phe Tyr Leu Ala Gly Asp Tyr Thr Lys Gln Lys Tyr Leu Ala Ser
           515                 520                 525
   Met Glu Gly Ala Val Leu Ser Gly Lys Phe Cys Ser Gln Ser Ile Val
       530                 535                 540
   Gln Asp Tyr Glu Leu Leu Ala Ala Ser Gly Pro Arg Lys Leu Ser Glu
   545                 550                 555                 560
   Ala Thr Val Ser Ser Ser
                   565
<210> 3
   <211> 1419
   <212> DNA
   <213> Synechocystis PCC6803
<400> 3
   atgcgcgttg tgatcgccgg agccggatta gccggcctag cctgtgccaa atacttagcc 60
   gatgcgggct ttacccccgt cgtcttggaa cgtagggatg tattaggcgg gaagatcgcc 120
   gcgtggaaag atgaggacgg agattggtac gaaaccggcc tacacatttt ttttggggcc 180
   tatcccaaca tgttgcagtt atttaaggaa ttggatatcg aagatcgtct gcaatggaaa 240
   gagcacagca tgatcttcaa ccaaccagag aaaccaggta cctactctcg gttcgatttt 300
   ccggatattc cggcccccat caatggtttg gtagccattc ttcgcaacaa cgatatgctt 360
   acctggccgg agaaaattcg ctttggcttg ggactcttgc cggccattgt ccagggccag 420
   agctatgtgg aagaaatgga taaatacact tggtcagagt ggatggccaa acaaaatatt 480
   cccccccgca tcgaaaaaga agttttcatt gccatgagta agacgttgaa ctttattgat 540
   cccgatgaaa tttccgccac cattttactt actgccctca atcgcttttt acaggaaaaa 600
   aatggctcta agatggcatt cctggatggg gcaccaccgg agcgtctttg ccaacctttg 660
   gtcgactata ttacggaacg gggaggggaa gtacacatta ataaacctct caaagaaatt 720
   ttgcttaatg aagatggttc cgttaagggt tacttaatcc ggggcctaga tggagccccc 780
   gacgaagtga tcacagcgga tttatatgtg tctgccatgc cggtggatcc cctgaaaacc 840
   atggtgccag cgccctggag agaatatcct gagtttaagc aaatccaagg tttggaagga 900
   gtcccggtca ttaacctcca cctgtggttt gaccgtaagt taaccgacat tgatcatttg 960
   ttattctccc gatcgccgtt gttgagtgtt tacgccgaca tgagcaacac ctgccgagaa 1020
   tacagtgatc cagacaaatc catgttggaa ttggtgctgg ctccggccca ggattggatc 1080
   ggcaaatccg acgaagagat tgtggcggcc accatggcgg agatcaagca actctttccc 1140
   caacacttca acggggataa tccagcccga ctgcttaaat cccacgtggt caaaaccccc 1200
   cgctcagtct acaaagctac ccccggaagg caggcttgtc gccccgatca acggacatcg 1260
   gtgcccaact tttacctagc aggggacttc accatgcaaa aatacttggg cagtatggaa 1320
   ggggcggtgc tttccggcaa acaatgcgcc caggcgatcg ccgccgattt caacccccaa 1380
   accgttcccc ccaccaggga aatagtcacc gtgggttaa                        1419
<210> 4
   <211> 472
   <212> PRT
   <213> Synechocystis PCC6803
<400> 4
   Met Arg Val Val Ile Ala Gly Ala Gly Leu Ala Gly Leu Ala Cys Ala
   1               5                   10                  15
   Lys Tyr Leu Ala Asp Ala Gly Phe Thr Pro Val Val Leu Glu Arg Arg
               20                  25                  30
   Asp Val Leu Gly Gly Lys Ile Ala Ala Trp Lys Asp Glu Asp Gly Asp
           35                  40                  45
   Trp Tyr Glu Thr Gly Leu His Ile Phe Phe Gly Ala Tyr Pro Asn Met
       50                  55                  60
   Leu Gln Leu Phe Lys Glu Leu Asp Ile Glu Asp Arg Leu Gln Trp Lys
   65                  70                  75                  80
   Glu His Ser Met Ile Phe Asn Gln Pro Glu Lys Pro Gly Thr Tyr Ser
                   85                  90                  95
   Arg Phe Asp Phe Pro Asp Ile Pro Ala Pro Ile Asn Gly Leu Val Ala
               100                 105                 110
   Ile Leu Arg Asn Asn Asp Met Leu Thr Trp Pro Glu Lys Ile Arg Phe
           115                 120                 125
   Gly Leu Gly Leu Leu Pro Ala Ile Val Gln Gly Gln Ser Tyr Val Glu
       130                 135                 140
   Glu Met Asp Lys Tyr Thr Trp Ser Glu Trp Met Ala Lys Gln Asn Ile
   145                 150                 155                 160
   Pro Pro Arg Ile Glu Lys Glu Val Phe Ile Ala Met Ser Lys Thr Leu
                   165                 170                 175
   Asn Phe Ile Asp Pro Asp Glu Ile Ser Ala Thr Ile Leu Leu Thr Ala
               180                 185                 190
   Leu Asn Arg Phe Leu Gln Glu Lys Asn Gly Ser Lys Met Ala Phe Leu
           195                 200                 205
   Asp Gly Ala Pro Pro Glu Arg Leu Cys Gln Pro Leu Val Asp Tyr Ile
       210                 215                 220
   Thr Glu Arg Gly Gly Glu Val His Ile Asn Lys Pro Leu Lys Glu Ile
   225                 230                 235                 240
   Leu Leu Asn Glu Asp Gly Ser Val Lys Gly Tyr Leu Ile Arg Gly Leu
                   245                 250                 255
   Asp Gly Ala Pro Asp Glu Val Ile Thr Ala Asp Leu Tyr Val Ser Ala
               260                 265                 270
   Met Pro Val Asp Pro Leu Lys Thr Met Val Pro Ala Pro Trp Arg Glu
           275                 280                 285
   Tyr Pro Glu Phe Lys Gln Ile Gln Gly Leu Glu Gly Val Pro Val Ile
       290                 295                 300
   Asn Leu His Leu Trp Phe Asp Arg Lys Leu Thr Asp Ile Asp His Leu
   305                 310                 315                 320
   Leu Phe Ser Arg Ser Pro Leu Leu Ser Val Tyr Ala Asp Met Ser Asn
                   325                 330                 335
   Thr Cys Arg Glu Tyr Ser Asp Pro Asp Lys Ser Met Leu Glu Leu Val
               340                 345                 350
   Leu Ala Pro Ala Gln Asp Trp Ile Gly Lys Ser Asp Glu Glu Ile Val
           355                 360                 365
   Ala Ala Thr Met Ala Glu Ile Lys Gln Leu Phe Pro Gln His Phe Asn
       370                 375                 380
   Gly Asp Asn Pro Ala Arg Leu Leu Lys Ser His Val Val Lys Thr Pro
   385                 390                 395                 400
   Arg Ser Val Tyr Lys Ala Thr Pro Gly Arg Gln Ala Cys Arg Pro Asp
                   405                 410                 415
   Gln Arg Thr Ser Val Pro Asn Phe Tyr Leu Ala Gly Asp Phe Thr Met
               420                 425                 430
   Gln Lys Tyr Leu Gly Ser Met Glu Gly Ala Val Leu Ser Gly Lys Gln
           435                 440                 445
   Cys Ala Gln Ala Ile Ala Ala Asp Phe Asn Pro Gln Thr Val Pro Pro
       450                 455                 460
   Thr Arg Glu Ile Val Thr Val Gly
   465                 470
<210> 5
   <211> 1698
   <212> DNA
   <213> Arabidopsis thaliana
<400> 5
   atggttgtgt ttgggaatgt ttctgcggcg aatttgcctt atcaaaacgg gtttttggag 60
   gcactttcat ctggaggttg tgaactaatg ggacatagct ttagggttcc cacttctcaa 120
   gcgcttaaga caagaacaag gaggaggagt actgctggtc ctttgcaggt agtttgtgtg 180
   gatattccaa ggccagagct agagaacact gtcaatttct tggaagctgc tagtttatct 240
   gcatccttcc gtagtgctcc tcgtcctgct aagcctttga aagttgtaat tgctggtgct 300
   ggattggctg gattgtcaac tgcaaagtac ctggctgatg caggccacaa acctctgttg 360
   cttgaagcaa gagatgttct tggtggaaag atagctgcat ggaaggatga agatggggac 420
   tggtatgaga ctggtttaca tatttttctc ggtgcttatc cgaatgtgca gaatttattt 480
   ggagaacttg ggatcaatga tcggttgcag tggaaggaac actccatgat ttttgctatg 540
   ccaagtaaac ctggagaatt tagtagattt gacttcccag atgtcctacc agcaccctta 600
   aatggtattt gggctatttt gcggaacaac gagatgctga catggccaga gaaaataaag 660
   tttgctattg gacttttgcc agccatggtc ggcggtcagg cttatgttga ggcccaagat 720
   ggtttatcag tcaaagaatg gatggaaaag cagggagtac ctgagcgcgt gaccgacgag 780
   gtgtttattg ccatgtcaaa gacgctaaac tttataaacc ctgatgaact gtcaatgcaa 840
   tgcattttga tagctttgaa cagctttctt caggaaaaac atggttccaa gatggcattc 900
   ttggatggta atcctccgga aaggctttgt atgccagtag tggatcatat tcgatcacta 960
   ggtggggaag tgcaacttaa ttctaggata aagaaaattg agctcaatga cgatggcacg 1020
   gttaagagtt tcttactcac taatggaagc actgtcgaag gagacgctta tgtgtttgcc 1080
   gctccagtcg atatcctgaa gctcctttta ccagatccct ggaaagaaat accgtacttc 1140
   aagaaattgg ataaattagt tggagtacca gttattaatg ttcatatatg gtttgatcga 1200
   aaactgaaga acacatatga tcacctactc tttagcagaa gtaaccttct gagcgtgtat 1260
   gccgacatgt ccttaacttg taaggaatat tacgatccta accggtcaat gctggagcta 1320
   gtatttgcac cagcagagga atggatatca cggactgatt ctgacatcat agatgcaaca 1380
   atgaaagaac ttgagaaact cttccctgat gaaatctcag ctgaccaaag caaagctaaa 1440
   attctgaagt accatgtcgt taagactcca agatctgtgt acaagaccat cccaaactgt 1500
   gaaccatgtc gtcctctaca aagatcacct attgaaggat tctacttagc tggagattac 1560
   acaaaacaga agtacttagc ttccatggaa ggcgctgtcc tctctggcaa attctgctct 1620
   cagtctattg ttcaggatta cgagctactg gctgcgtctg gaccaagaaa gttgtcggag 1680
   gcaacagtat catcatca                                               1698
<210> 6
   <211> 566
   <212> PRT
   <213> Arabidopsis thaliana
<400> 6
   Met Val Val Phe Gly Asn Val Ser Ala Ala Asn Leu Pro Tyr Gln Asn
   1               5                   10                  15
   Gly Phe Leu Glu Ala Leu Ser Ser Gly Gly Cys Glu Leu Met Gly His
               20                  25                  30
   Ser Phe Arg Val Pro Thr Ser Gln Ala Leu Lys Thr Arg Thr Arg Arg
           35                  40                  45
   Arg Ser Thr Ala Gly Pro Leu Gln Val Val Cys Val Asp Ile Pro Arg
       50                  55                  60
   Pro Glu Leu Glu Asn Thr Val Asn Phe Leu Glu Ala Ala Ser Leu Ser
   65                  70                  75                  80
   Ala Ser Phe Arg Ser Ala Pro Arg Pro Ala Lys Pro Leu Lys Val Val
                   85                  90                  95
   Ile Ala Gly Ala Gly Leu Ala Gly Leu Ser Thr Ala Lys Tyr Leu Ala
               100                 105                 110
   Asp Ala Gly His Lys Pro Leu Leu Leu Glu Ala Arg Asp Val Leu Gly
           115                 120                 125
   Gly Lys Ile Ala Ala Trp Lys Asp Glu Asp Gly Asp Trp Tyr Glu Thr
       130                 135                 140
   Gly Leu His Ile Phe Phe Gly Ala Tyr Pro Asn Val Gln Asn Leu Phe
   145                 150                 155                 160
   Gly Glu Leu Gly Ile Asn Asp Arg Leu Gln Trp Lys Glu His Ser Met
                   165                 170                 175
   Ile Phe Ala Met Pro Ser Lys Pro Gly Glu Phe Ser Arg Phe Asp Phe
               180                 185                 190
   Pro Asp Val Leu Pro Ala Pro Leu Asn Gly Ile Trp Ala Ile Leu Arg
           195                 200                 205
   Asn Asn Glu Met Leu Thr Trp Pro Glu Lys Ile Lys Phe Ala Ile Gly
       210                 215                 220
   Leu Leu Pro Ala Met Val Gly Gly Gln Ala Tyr Val Glu Ala Gln Asp
   225                 230                 235                 240
   Gly Leu Ser Val Lys Glu Trp Met Glu Lys Gln Gly Val Pro Glu Arg
                   245                 250                 255
   Val Thr Asp Glu Val Phe Ile Ala Met Ser Lys Thr Leu Asn Phe Ile
               260                 265                 270
   Asn Pro Asp Glu Leu Ser Met Gln Cys Ile Leu Ile Ala Leu Asn Ser
           275                 280                 285
   Phe Leu Gln Glu Lys His Gly Ser Lys Met Ala Phe Leu Asp Gly Asn
       290                 295                 300
   Pro Pro Glu Arg Leu Cys Met Pro Val Val Asp His Ile Arg Ser Leu
   305                 310                 315                 320
   Gly Gly Glu Val Gln Leu Asn Ser Arg Ile Lys Lys Ile Glu Leu Asn
                   325                 330                 335
   Asp Asp Gly Thr Val Lys Ser Phe Leu Leu Thr Asn Gly Ser Thr Val
               340                 345                 350
   Glu Gly Asp Ala Tyr Val Phe Ala Ala Pro Val Asp Ile Leu Lys Leu
           355                 360                 365
   Leu Leu Pro Asp Pro Trp Lys Glu Ile Pro Tyr Phe Lys Lys Leu Asp
       370                 375                 380
   Lys Leu Val Gly Val Pro Val Ile Asn Val His Ile Trp Phe Asp Arg
   385                 390                 395                 400
   Lys Leu Lys Asn Thr Tyr Asp His Leu Leu Phe Ser Arg Ser Asn Leu
                   405                 410                 415
   Leu Ser Val Tyr Ala Asp Met Ser Leu Thr Cys Lys Glu Tyr Tyr Asp
               420                 425                 430
   Pro Asn Arg Ser Met Leu Glu Leu Val Phe Ala Pro Ala Glu Glu Trp
           435                 440                 445
   Ile Ser Arg Thr Asp Ser Asp Ile Ile Asp Ala Thr Met Lys Glu Leu
       450                 455                 460
   Glu Lys Leu Phe Pro Asp Glu Ile Ser Ala Asp Gln Ser Lys Ala Lys
   465                 470                 475                 480
   Ile Leu Lys Tyr His Val Val Lys Thr Pro Arg Ser Val Tyr Lys Thr
                   485                 490                 495
   Ile Pro Asn Cys Glu Pro Cys Arg Pro Leu Gln Arg Ser Pro Ile Glu
               500                 505                 510
   Gly Phe Tyr Leu Ala Gly Asp Tyr Thr Lys Gln Lys Tyr Leu Ala Ser
           515                 520                 525
   Met Glu Gly Ala Val Leu Ser Gly Lys Phe Cys Ser Gln Ser Ile Val
       530                 535                 540
   Gln Asp Tyr Glu Leu Leu Ala Ala Ser Gly Pro Arg Lys Leu Ser Glu
   545                 550                 555                 560
   Ala Thr Val Ser Ser Ser
                   565
<210> 7
   <211> 1698
   <212> DNA
   <213> Arabidopsis thaliana
<400> 7
   atggttgtgt ttgggaatgt ttctgcggcg aatttgcctt atcaaaacgg gtttttggag 60
   gcactttcat ctggaggttg tgaactaatg ggacatagct ttagggttcc cacttctcaa 120
   gcgcttaaga caagaacaag gaggaggagt actgctggtc ctttgcaggt agtttgtgtg 180
   gatattccaa ggccagagct agagaacact gtcaatttct tggaagctgc tagtttatct 240
   gcatccttcc gtagtgctcc tcgtcctgct aagcctttga aagttgtaat tgctggtgct 300
   ggattggctg gattgtcaac tgcaaagtac ctggctgatg caggccacaa acctctgttg 360
   cttgaagcaa gagatgttct tggtggaaag atagctgcat ggaaggatga agatggggac 420
   tggtatgaga ctggtttaca tattttcttc ggtgcttatc cgaatgtgca gaatttattt 480
   ggagaacttg ggatcaatga tcggttgcag tggaaggaac actccatgat ttttgctatg 540
   ccaagtaaac ctggagaatt tagtagattt gacttcccag atgtcctacc agcaccctta 600
   aatggtattt gggctatttt gcggaacaac gagatgctga catggccaga gaaaataaag 660
   tttgctattg gacttttgcc agccatggtc ggcggtcagg cttatgttga ggcccaagat 720
   ggtttatcag tcaaagaatg gatggaaaag cagggagtac ctgagcgcgt gaccgacgag 780
   gtgtttattg ccatgtcaaa gacgctaaac tttataaacc ctgatgaact gtcaatgcaa 840
   tgcattttga tagctttgaa ccggtttctt caggaaaaac atggttccaa gatggcattc 900
   ttggatggta atcctccgga aaggctttgt atgccagtag tggatcatat tcgatcacta 960
   ggtggggaag tgcaacttaa ttctaggata aagaaaattg agctcaatga cgatggcacg 1020
   gttaagagtt tcttactcac taatggaagc actgtcgaag gagacgctta tgtgtttgcc 1080
   gctccagtcg atatcctgaa gctcctttta ccagatccct ggaaagaaat accgtacttc 1140
   aagaaattgg ataaattagt tggagtacca gttattaatg ttcatatatg gtttgatcga 1200
   aaactgaaga acacatatga tcacctactc tttagcagaa gtaaccttct gagcgtgtat 1260
   gccgacatgt ccttaacttg taaggaatat tacgatccta accggtcaat gctggagcta 1320
   gtatttgcac cagcagagga atggatatca cggactgatt ctgacatcat agatgcaaca 1380
   atgaaagaac ttgagaaact cttccctgat gaaatctcag ctgaccaaag caaagctaaa 1440
   attctgaagt accatgtcgt taagactcca agatctgtgt acaagaccat cccaaactgt 1500
   gaaccatgtc gtcctctaca aagatcacct attgaaggat tctacttagc tggagattac 1560
   acaaaacaga agtacttagc ttccatggaa ggcgctgtcc tctctggcaa attctgctct 1620
   cagtctattg ttcaggatta cgagctactg gctgcgtctg gaccaagaaa gttgtcggag 1680
   gcaacagtat catcatca                                                1698
<210> 8
   <211> 566
   <212> PRT
   <213> Arabidopsis thaliana
<400> 8
   Met Val Val Phe Gly Asn Val Ser Ala Ala Asn Leu Pro Tyr Gln Asn
   1               5                   10                  15
   Gly Phe Leu Glu Ala Leu Ser Ser Gly Gly Cys Glu Leu Met Gly His
               20                  25                  30
   Ser Phe Arg Val Pro Thr Ser Gln Ala Leu Lys Thr Arg Thr Arg Arg
           35                  40                  45
   Arg Ser Thr Ala Gly Pro Leu Gln Val Val Cys Val Asp Ile Pro Arg
       50                  55                  60
   Pro Glu Leu Glu Asn Thr Val Asn Phe Leu Glu Ala Ala Ser Leu Ser
   65                  70                  75                  80
   Ala Ser Phe Arg Ser Ala Pro Arg Pro Ala Lys Pro Leu Lys Val Val
                   85                  90                  95
   Ile Ala Gly Ala Gly Leu Ala Gly Leu Ser Thr Ala Lys Tyr Leu Ala
               100                 105                 110
   Asp Ala Gly His Lys Pro Leu Leu Leu Glu Ala Arg Asp Val Leu Gly
           115                 120                 125
   Gly Lys Ile Ala Ala Trp Lys Asp Glu Asp Gly Asp Trp Tyr Glu Thr
       130                 135                 140
   Gly Leu His Ile Phe Phe Gly Ala Tyr Pro Asn Val Gln Asn Leu Phe
   145                 150                 155                 160
   Gly Glu Leu Gly Ile Asn Asp Arg Leu Gln Trp Lys Glu His Ser Met
                   165                 170                 175
   Ile Phe Ala Met Pro Ser Lys Pro Gly Glu Phe Ser Arg Phe Asp Phe
               180                 185                 190
   Pro Asp Val Leu Pro Ala Pro Leu Asn Gly Ile Trp Ala Ile Leu Arg
           195                 200                 205
   Asn Asn Glu Met Leu Thr Trp Pro Glu Lys Ile Lys Phe Ala Ile Gly
       210                 215                 220
   Leu Leu Pro Ala Met Val Gly Gly Gln Ala Tyr Val Glu Ala Gln Asp
   225                 230                 235                 240
   Gly Leu Ser Val Lys Glu Trp Met Glu Lys Gln Gly Val Pro Glu Arg
                   245                 250                 255
   Val Thr Asp Glu Val Phe Ile Ala Met Ser Lys Thr Leu Asn Phe Ile
               260                 265                 270
   Asn Pro Asp Glu Leu Ser Met Gln Cys Ile Leu Ile Ala Leu Asn Arg
           275                 280                 285
   Phe Leu Gln Glu Lys His Gly Ser Lys Met Ala Phe Leu Asp Gly Asn
       290                 295                 300
   Pro Pro Glu Arg Leu Cys Met Pro Val Val Asp His Ile Arg Ser Leu
   305                 310                 315                 320
   Gly Gly Glu Val Gln Leu Asn Ser Arg Ile Lys Lys Ile Glu Leu Asn
                   325                 330                 335
   Asp Asp Gly Thr Val Lys Ser Phe Leu Leu Thr Asn Gly Ser Thr Val
               340                 345                 350
   Glu Gly Asp Ala Tyr Val Phe Ala Ala Pro Val Asp Ile Leu Lys Leu
           355                 360                 365
   Leu Leu Pro Asp Pro Trp Lys Glu Ile Pro Tyr Phe Lys Lys Leu Asp
       370                 375                 380
   Lys Leu Val Gly Val Pro Val Ile Asn Val His Ile Trp Phe Asp Arg
   385                 390                 395                 400
   Lys Leu Lys Asn Thr Tyr Asp His Leu Leu Phe Ser Arg Ser Asn Leu
                   405                 410                 415
   Leu Ser Val Tyr Ala Asp Met Ser Leu Thr Cys Lys Glu Tyr Tyr Asp
               420                 425                 430
   Pro Asn Arg Ser Met Leu Glu Leu Val Phe Ala Pro Ala Glu Glu Trp
           435                 440                 445
   Ile Ser Arg Thr Asp Ser Asp Ile Ile Asp Ala Thr Met Lys Glu Leu
       450                 455                 460
   Glu Lys Leu Phe Pro Asp Glu Ile Ser Ala Asp Gln Ser Lys Ala Lys
   465                 470                 475                     480
   Ile Leu Lys Tyr His Val Val Lys Thr Pro Arg Ser Val Tyr Lys Thr
                   485                 490                 495
   Ile Pro Asn Cys Glu Pro Cys Arg Pro Leu Gln Arg Ser Pro Ile Glu
               500                 505                 510
   Gly Phe Tyr Leu Ala Gly Asp Tyr Thr Lys Gln Lys Tyr Leu Ala Ser
           515                 520                 525
   Met Glu Gly Ala Val Leu Ser Gly Lys Phe Cys Ser Gln Ser Ile Val
       530                 535                 540
   Gln Asp Tyr Glu Leu Leu Ala Ala Ser Gly Pro Arg Lys Leu Ser Glu
   545                 550                 555                     560
   Ala Thr Val Ser Ser Ser
                   565

## Description

### BACKGROUND OF THE INVENTION

### Field of the Invention

This invention relates generally to polynucleotide sequences encoding proteins that are associated with herbicide resistance in plants. In particular, this invention relates to polynucleotide sequences encoding proteins that increase carotenoid biosynthesis inhibitor (CBI) resistance in plants.

### Background Art

Carotenoids are essential protective pigments that are synthesized by all photosynthetic organisms, including plants, algae, and cyanobacteria, as well as many nonphotosynthetic bacteria and fungi (Cunningham & Grant, Ann. Rev. Plant Physiol. Plant Mol. Biol. (1998) 49:557-83). Carotenoids are synthesized as either hydrocarbons (carotenes) or oxygenated derivatives (xanthophylls). Carotenoids have several functions, such as contributing to the stability of certain complexes in photosynthesis and harvesting light in the blue-green region of the spectrum, transferring it to chlorophyll. A particularly important function of the carotenoids is the protection of cells and organisms from photooxidative damage by absorbing light energy.

Several inhibitors of the carotenoid biosynthesis pathway have been developed for commercial use as herbicides over the last two decades (Bramley, Carotenoid Biosynthesis in Target Sites for Herbicide Action (1991) pp. 95-122). For example, fluridone, norflurazon, AC 375488, and AC 900001 are all used as bleaching herbicides which target the enzymes of the carotenoid biosynthesis pathway. The principal target of the majority of CBI compounds is phytoene desaturase (PDS), the first enzyme involved in the conversion of phytoene to colored carotenoids. In plants, PDS is a single-gene membrane-bound enzyme that catalyzes two sequential desaturation reactions that take place in the chloroplast membrane. PDS utilizes the identical halves of symmetrical phytoene as a substrate to produce ζ-carotene, with phytofluene as an intermediate. When a plant is treated with a CBI compound that targets PDS, the plant accumulates phytoene, and bleaching of the plant occurs (Chamovitz et al., Plant Mol. Biol. (1991) 16:967-74). Bleaching herbicides such as norflurazon and fluridone act as non-competitive inhibitors of PDS by direct interference (Misawa et al., Plant J. (1993) 4(5):833-40).

Weed control is necessary for the efficient production of commercial crops. Therefore, it would be beneficial to have plants with increased resistance to certain compounds, such as CBI compounds. Herbicides interfering with carotenoid biosynthesis cause decreased levels of colored carotenoids in the plant, leading to photooxidation of chlorophyll and limited assembly of the photosynthetic apparatus in the plant, thereby decreasing the plant's photosynthetic activity. Further, a decreased level of colored carotenoids results in a decrease of the protective function of these pigments. Photobleaching by oxidative degradation of chlorophyll and destruction of the photosynthetic membranes are a direct result. CBI compounds cause bleaching of only the growing leaves and not the non-growing parts of the leaf. Accordingly, these compounds are essentially pre-emergent or post-emergent herbicides.

CBI herbicide AC 375488 demonstrates excellent potential for pre-emergence stand alone cross spectrum weed control in winter wheat. The weeds controlled include the most difficult to control weeds in European cereals, *Alopecurus myosuroides,* and *Galium aparine.* CBI herbicide AC 375488 has shown season long control of all major broadleaf weeds. Another advantage of CBI herbicides is that mammals do not have this biosynthetic pathway. Accordingly, mammals are less likely to be harmed by these herbicides. In fact, no toxicological problems have been identified based on the limited current data regarding CBI herbicide AC 375488. By treating plants that have increased CBI resistance with a CBI compound as either a pre-emergent or post-emergent spray, the surrounding weeds would be controlled, reducing competition for nutrients, without harming the plants that have increased CBI resistance.

Polynucleotide sequences that increase resistance to CBI compounds have been identified in two cyanobacterial species, a bacterium, and a fungus. For example, a mutant strain of the cyanobacterium *Synechococcus* PCC7942 that has increased CBI resistance was isolated (Chamovitz et al., Plant Mol. Biol. (1991) 16:967-74). The increased CBI resistance was found to be the result of a single point mutation that resulted in a valine to glycine amino acid substitution at position 403 of PDS. Similarly, a polynucleotide sequence that increases resistance to CBI compounds has been identified in another cyanobacterium *Synechocystis* PCC6803 (Martinez-Ferez & Vioque, Plant Mol. Biol. (1992) 18:981-83). The cyanobacterium with increased resistance to CBI compounds contained an arginine to cysteine amino acid substitution at position 195 of PDS.

There are also certain organisms that are naturally resistant to CBI compounds. For example, the plant fungal pathogen *Cercospora nicotianae* is believed to naturally have increased resistance to the action of CBI compounds (Ehrenshaft & Daub, Appl. Env. Microbiol. (1994) 2766-71). Ehrenshaft and Daub demonstrated the CBI resistance of *Cercospora nicotianae* and isolated and sequenced the polynucleotide that encodes phytoene dehydrogenase (PDH), an enzyme in the carotenoid biosynthesis pathway. In addition, the plant bacterial pathogen *Erwinia uredovora* has increased CBI resistance. Misawa et al. demonstrated that introducing the *E*. *uredovora crtI* polynucleotide that encodes PDS into a tobacco plant increases the tobacco plant's CBI resistance (Misawa et al., The Plant J. (1993) 4(5):833-840).

What is needed is the identification of additional polynucleotides and proteins involved in increased resistance to a CBI compound. Identification of additional polynucleotides, or mutations in polynucleotides, that increase CBI resistance in plants will not only advance the understanding of plant adaptation and resistance to bleaching herbicides, but also may provide important information for designing new strategies for crop improvement.

A common concern with regard to the introduction of herbicide resistance genes into plants is that the foreign gene might escape by means of pollen dispersal from the transgenic plants to neighboring weeds (Umbeck et al., Journal of Econ. Entomol (1991) 84(6): 1943-50; Lewellyn & Fitt, Molecular Breeding (1996) 2:157-66). To avoid this problem, the desired herbicide resistance genes may be introduced into the chloroplast genome via plastid transformation. Because the chloroplasts are maternally inherited in most crops, targeting the genes to the chloroplast genome would prevent gene escape through pollen (Daniell & McFadden, Proc. Nat'l Acad. Sci. (1987) 84:6349-53; Boynton et al., Science (1988) 24:1534-38; Daniell et al., Proc. Nat'l Acad. Sci. (1990) 87:88-92; Svab et al., Proc. Nat'l Acad. Sci. (1990) 87:8526-30; Daniell et al., Plant Cell Reports (1991) 9:615-19; Svab & Maliga, Proc. Nat'l Acad. Sci. (1993) 90:913-17; Zoubenko et al., Nucleic Acids Research (1994) 22(19):3819-24; Daniell et al., Nature Biotech. (1998) 16:345-48;). In addition, the very high copy number of chloroplast genomes in plant cells allows for very high levels of expression of the foreign gene (Bendich, Bioessays (1987) 6(6):279-82; Daniell et al., Nature Biotech. (1998) 16:345-48). A distinct advantage of plastid transformation is that bacterial genes conferring herbicide resistance can easily be expressed in plants because the transcriptional and translational machinery of the chloroplast is prokaryotic in nature.

There is a need, therefore, to identify polynucleotides that have the capacity to increase CBI resistance in its host organism and in other plant species. There is a need for plants with increased resistance to a CBI compound such as fluridone, norflurazon, AC 375488, and AC 900001, and methods for controlling weed growth in the vicinity of the CBI resistant plants. There is also a need to introduce CBI resistance in plants without the possibility of unwanted transfer of the transgenes to neighboring weeds through pollen dispersal.

### SUMMARY OF THE INVENTION

This invention fulfills in part the need to identify new, unique polynucleotides and proteins capable of increasing herbicide resistance in plants. The present invention provides a transgenic plant having increased resistance to a Carotenoid Biosynthesis Inhibitor (CBI) compound as compared to a wild type variety of the plant. In particular, the present invention relates to plants comprising a Carotenoid Biosynthesis Inhibitor Resistant Protein (CBIRP) coding nucleic acid, wherein expression of the nucleic acid in the plant results in increased resistance to a CBI compound as compared to a wild type variety of the plant. The invention provides that the CBI compound can be, for example, fluridone, norflurazon, AC 375488, and AC900001, or combinations thereof.

The invention provides a transgenic plant comprising a CBIRP coding nucleic acid, wherein the plant is true breeding for increased resistance to a CBI compound as compared to a wild type variety of the plant. The present invention also provides a transgenic plant cell comprising a CBIRP coding nucleic acid. The invention also provides a seed produced by a transgenic plant comprising a CBIRP coding nucleic acid, wherein the plant is true breeding for increased resistance to a CBI compound as compared to a wild type variety of the plant. The invention further provides a seed produced by a transgenic plant expressing a CBIRP, wherein the plant is true breeding for increased CBI resistance as compared to a wild type variety of the plant. The invention further provides an agricultural product produced by any of the below-described transgenic plants, plant parts, or seeds.

The invention further provides an isolated CBIRP and an isolated CBIRP coding nucleic acid as described below. Namely, described herein are CBIRP coding nucleic acids wherein the CBIRP is selected from the group consisting of: 1) a phytoene desaturase (PDS) from *Arabidopsis thaliana* and 2) a PDS from *Synechocystis* PCC6803. The invention provides in some embodiments that the CBIRP and CBIRP coding nucleic acid are from *Arabidopsis thaliana.* The invention describes that the CBIRP and CBIRP coding nucleic acid are from *Synechocystis* PCC6803.

The invention also provides an isolated recombinant expression vector comprising a CBIRP coding nucleic acid, wherein expression of the vector in a host cell results in increased resistance to a CBI compound as compared to a wild type variety of the host cell. The invention provides that the expression vector may target either the nucleus or the chloroplast of the host cell. The invention further provides a host cell containing the vector and a plant containing the host cell.

The invention further provides a method for producing a transgenic plant containing a CBIRP coding nucleic acid, wherein expression of the nucleic acid in the plant results in the transgenic plant's increased CBI resistance as compared to a wild type variety of the plant comprising: (a) transforming a plant cell with a nuclear or plastid expression vector comprising a CBIRP coding nucleic acid, and (b) generating from the plant cell the transgenic plant with an increased resistance to a CBI compound as compared to a wild type variety of the plant. Namely, described herein is a method for producing a transgenic plant containing a CBIRP coding nucleic acid, wherein expression of the nucleic acid in the plant results in the transgenic plant's increased CBI resistance, and wherein the CBIRP is selected from the group consisting of: 1) a phytoene desaturase (PDS) from *Arabidopsis thaliana*; and 2) a PDS from *Synechocystis* PCC6803.

In another aspect, the invention provides methods for controlling weeds in the vicinity of a transgenic plant, comprising applying a CBI compound to the weeds and to the transgenic plant, wherein the plant has increased resistance to the CBI compound as compared to a wild type variety of the plant.

The present invention further describes a method for identifying a novel CBIRP, comprising (a) treating one or more organisms with a CBI compound, (b) screening for an organism that has increased resistance to the CBI compound as demonstrated by the organisms surviving the treatment with the CBI compound, and (c) identifying a mutation that contributed to the increased CBI resistance. Alternatively, the present invention describes another method for identifying a novel CBIRP, comprising (a) performing EMS mutagenesis of one or more organisms to introduce random mutations throughout the genome, (b) treating the one or more organisms with a CBI compound, (c) screening for an organism with increased CBI resistance as indicated by the organisms surviving the treatment with the CBI compound, and (d) identifying a mutation that contributed to the increased resistance to the CBI compound.

### BRIEF DESCRIPTION OF THE DRAWINGS

Figure 1 is a graph showing the increase in tolerance of transgenic tobacco plants obtained following nuclear transformation with plasmid pACBC1001 containing the *Arabidopsis thaliana* R288S PDS gene to norflurazon as compared to the control wild type tobacco plants.

Figure 2 is a graph showing the increase in tolerance of transgenic tobacco plants obtained following plastid transformation with plasmid p121-I containing the *Arabidopsis thaliana* R288P PDS gene to norflurazon as compared to the control wild type tobacco plants.

### DETAILED DESCRIPTION OF THE INVENTION

The present invention may be understood more readily by reference to the following detailed description of the preferred embodiments of the invention and the Examples included herein. In particular, the designation of the amino acid sequences as "Carotenoid Biosynthesis Inhibitor Resistant Proteins (CBIRPs)," in no way limits the functionality of those sequences.

The present invention describes a novel genus of CBIRPs and CBIRP coding nucleic acids that are important for modulating a plant's response to a CBI compound. More particularly, expression of these CBIRP coding nucleic acids in a plant results in the plant's increased resistance to a CBI compound.

The present invention provides a transgenic plant cell containing a CBIRP coding nucleic acid, wherein expression of the nucleic acid in the plant cell results in increased resistance to a CBI compound as compared to a wild type variety of the plant cell. The invention further provides transgenic plants and transgenic plant parts containing the plant cells, wherein the transgenic plants and plant parts have increased resistance to a CBI compound as compared to a wild type variety of the plant or plant part. Plant parts include, but are not limited to, stems, roots, ovules, stamens, leaves, embryos, meristematic regions, callus tissue, gametophytes, sporophytes, pollen, microspores and the like. In one embodiment, the transgenic plant is male sterile.

Also provided is a plant seed produced by a transgenic plant containing a CBIRP coding nucleic acid, wherein the seed contains the CBIRP coding nucleic acid, and wherein the plant is true breeding for increased CBI resistance as compared to a wild type variety of the plant. The invention further provides a seed produced by a transgenic plant expressing a CBIRP, wherein the seed contains the CBIRP, and wherein the plant is true breeding for increased CBI resistance as compared to a wild type variety of the plant. The invention also provides an agricultural product produced by any of the below-described transgenic plants, plant parts, and plant seeds. Agricultural products include, but are not limited to, plant extracts, proteins, amino acids, carbohydrates, fats, oils, polymers, vitamins, and the like.

As used herein, the term "variety" refers to a group of plants within a species that share constant characteristics that separate them from the typical form and from other possible varieties within that species. While possessing at least one distinctive trait, a variety is also characterized by some variation between individuals within the variety, based primarily on the Mendelian segregation of traits among the progeny of succeeding generations. A variety is considered "true breeding" for a particular trait if it is genetically homozygous for that trait to the extent that, when the true-breeding variety is self-pollinated, a significant amount of independent segregation of the trait among the progeny is not observed. In the present invention, the trait arises from the transgenic expression of one or more polynucleotide sequences introduced into a plant variety.

The present invention describes for the first time that altered PDS polypeptides from *Arabidopsis thaliana* and *Synechocystis* PCC6803 are useful for increasing a plant's resistance to a CBI compound. As used herein, the term "polypeptide," is used interchangeably with the term "protein" and refers to a chain of at least four amino acids joined by peptide bonds. The chain may be linear, branched, circular or combinations thereof. Accordingly, the present invention provides isolated CBIRPs which is a PDS protein from an *Arabidopsis* species, and homologs thereof.

In one embodiment of the present invention, the plant having increased resistance to a CBI compound comprises an altered PDS protein. As used herein, the term "altered PDS protein" refers to a PDS protein that is mutated from the wild type PDS protein and that increases CBI resistance in a plant in which it is expressed. The invention describes that the altered PDS protein comprises an arginine to serine, arginine to proline, or arginine to cysteine amino acid substitution at a position corresponding to position 288 of a wild type PDS protein from *Arabidopsis thaliana*. In a preferred embodiment, the altered PDS protein comprises an arginine to proline amino acid substitution at a position corresponding to position 288 of a wild type PDS protein from *Arabidopsis thaliana.* In a still further preferred embodiment, the altered PDS protein comprises a sequence as defined in SEQ ID NO:2. It is envisaged that the altered PDS protein comprises an alanine to threonine amino acid substitution at a position corresponding to position 268 of a wild type PDS protein from *Arabidopsis thaliana* and the altered PDS protein comprises a sequence as defined in SEQ ID NO:8. It is also envisaged that the altered PDS protein comprises an arginine to serine amino acid substitution at position 288 and an alanine to threonine substitution at Position 268 of a wild type PDS protein from *Arabidopsis thaliana* and the altered PDS protein comprises a sequence as defined in SEQ ID NO:6.

The invention describes that the altered PDS protein comprises an alanine to threonine amino acid substitution at a position corresponding to position 175 of a wild type PDS protein from *Synechocystis* PCC6803 and the altered PDS protein comprises a sequence as defined in SEQ ID NO:4.

Accordingly, in preferred embodiments, the plant having increased CBI resistance comprises a CBIRP selected from an altered PDS protein from *Arabidopsis thaliana* as defined in SEQ ID NO:2, and homologs and orthologs thereof. Homologs and orthologs of the amino acid sequences are defined below.

The CBIRPs of the present invention are preferably produced by recombinant DNA techniques. For example, a nucleic acid molecule encoding the polypeptide is cloned into an expression vector resulting in a recombinant polynucleotide (as described below), the expression vector is introduced into a host cell (as described below), and the CBIRP is expressed in the host cell. The CBIRP can then be isolated from the cells by an appropriate purification scheme using standard polypeptide purification techniques. For the purposes of the invention, the term "recombinant polynucleotide" refers to a polynucleotide that has been altered, rearranged or modified by genetic engineering. Examples include any cloned polynucleotide, and polynucleotides that are linked or joined to heterologous sequences. Alternative to recombinant expression, a CBIRP or polypeptide can be synthesized chemically using standard peptide synthesis techniques. Moreover, a CBIRP can be isolated from cells, for example, using an anti-CBIRP antibody, which can be produced by standard techniques utilizing a CBIRP or fragment thereof.

The invention further provides an isolated CBIRP coding nucleic acid. In one embodiment of the present invention, the plant having increased resistance to a CBI compound comprises an altered PDS nucleic acid. As used herein, the terms "altered PDS nucleic acid" and "altered PDS polynucleotide" refer to a PDS nucleic acid that is mutated from a wild type PDS nucleic acid and that increases CBI resistance in a plant in which it is expressed. In a preferred embodiment, the altered PDS nucleic acid comprises a two base pair substitution (guanine guanine to cytosine cytosine) at positions corresponding to 863 and 864 within the coding region in a wild type PDS nucleic acid from *Arabidopsis thaliana.* In a further preferred embodiment, the altered PDS nucleic acid comprises a polynucleotide sequence as defined in SEQ ID NO:1. The invention describes that the plant having increased resistance to a CBI compound comprises an altered *A. thaliana* PDS nucleic acid that encodes a polypeptide having an alanine to threonine substitution at position 268 of the wild type PDS polypeptide. and the PDS nucleic acid comprises the nucleotide sequence as defined in SEQ ID NO:7. It is also described that the plant having increased resistance to a CBI compound comprises an altered *A. thaliana* PDS nucleic acid that encodes a polypeptide having an alanine to threonine amino acid substitution at position 268 and an arginine to serine amino acid substitution at position 288 and the PDS nucleic acid comprises the nucleotide sequence as defined in SEQ ID NO:5.

It is envisaged that the altered PDS nucleic acid comprises a guanine to adenine base pair substitution at a position corresponding to position 523 in a wild type PDS nucleic acid from *Synechocystis* PCC6803 and the altered PDS nucleic acid comprises a polynucleotide sequence as defined in SEQ ID NO:3.

Accordingly, in preferred embodiments, the plant having increased resistance to a CBI compound contains a CBIRP coding nucleic acid comprising a polynucleotide sequence selected from an altered PDS polynucleotide from *Arabidopsis thaliana* as defined in SEQ ID NO:1, and homologs and orthologs thereof. Homologs and orthologs of the nucleotide sequences are defined bellow.

As used herein, the term "CBI compound" refers to any compound that inhibits the carotenoid biosynthesis pathway and includes, but is not limited to, fluridone, phenylpyridazinones (e.g. norflurazon), AC 375488, AC 900001, metflurazon, phenylfuranones (e.g. difunone and derivatives), methoxybenzophenone, fluorochloridone, haloxydine, CGA 22867, phenoxybenzamides (e.g. S-3422), phenoxynicotinamides (e.g. diflufenican), flurtamone, diphenylpyrrolidinones (e.g. MTP1), tetrahydropyrimidinones (e.g. NTN 28621), ketomorpholines, diphenylpyridines, and combinations thereof (Boger and Sandman, 1998, Pesticide Outlook, 9:29-35). It is also to be understood that as used in the specification and in the claims, "a" or "an" can mean one or more, depending upon the context in which it is used. Thus, for example, reference to "a cell" can mean that at least one cell can be utilized.

As also used herein, the terms "nucleic acid" and polynucleotide" refer to RNA or DNA that is linear or branched, single or double stranded, or a hybrid thereof. These terms encompass RNA/DNA hybrids. These terms also encompass untranslated sequence located at both the 3' and 5' ends of the coding region of the gene: at least about 1000 nucleotides of sequence upstream from the 5' end of the coding region and at least about 200 nucleotides of sequence downstream from the 3' end of the coding region of the gene. Less common bases, such as inosine, 5-methylcytosine, 6-methyladenine, hypoxanthine and others can also be used for antisense, dsRNA and ribozyme pairing. For example, polynucleotides that contain C-5 propyne analogues of uridine and cytidine have been shown to bind RNA, with high affinity and to be potent antisense inhibitors of gene expression. Other modifications, such as modifications to the phosphodiester backbone, or the 2'-hydroxy in the ribose sugar group of the RNA, can also be made. The antisense polynucleotides and ribozymes can consist entirely of ribonucleotides, or can contain mixed ribonucleotides and deoxyribonucleotides. The polynucleotides of the invention may be produced by any means, including genomic preparations, cDNA preparations, *in vitro* synthesis, RT-PCR and *in vitro* or *in vivo* transcription.

An "isolated" nucleic acid molecule is one that is substantially separated from other nucleic acid molecules which are present in the natural source of the nucleic acid (i.e., sequences encoding other polypeptides). Preferably, an "isolated" nucleic acid is free of some of the sequences which naturally flank the nucleic acid (i.e., sequences located at the 5' and 3' ends of the nucleic acid) in its naturally occurring replicon. For example, a cloned nucleic acid is considered isolated. In various embodiments, the isolated CBIRP coding nucleic acid molecule can contain less than about 5 kb, 4 kb, 3 kb, 2 kb, 1 kb, 0.5 kb or 0.1 kb of nucleotide sequences which naturally flank the nucleic acid molecule in genomic DNA of the cell from which the nucleic acid is derived. A nucleic acid is also considered isolated if it has been altered by human intervention, or placed in a locus or location that is not its natural site, or if it is introduced into a cell by agroinfection. Moreover, an "isolated" nucleic acid molecule, such as a cDNA molecule, can be free from some of the other cellular material with which it is naturally associated, or culture medium when produced by recombinant techniques, or chemical precursors or other chemicals when chemically synthesized.

Specifically excluded from the definition of "isolated nucleic acids" are: naturally-occurring chromosomes (such as chromosome spreads), artificial chromosome libraries, genomic libraries, and cDNA libraries that exist either as an *in vitro* nucleic acid preparation or as a transfected/transformed host cell preparation, wherein the host cells are either an *in vitro* heterogeneous preparation or plated as a heterogeneous population of single colonies. Also specifically excluded are the above libraries wherein a specified nucleic acid makes up less than 5% of the number of nucleic acid inserts in the vector molecules. Further specifically excluded are whole cell genomic DNA or whole cell RNA preparations (including whole cell preparations that are mechanically sheared or enzymatically digested). Even further specifically excluded are the whole cell preparations found as either an *in vitro* preparation or as a heterogeneous mixture separated by electrophoresis wherein the nucleic acid of the invention has not further been separated from the heterologous nucleic acids in the electrophoresis medium (e.g., further separating by excising a single band from a heterogeneous band population in an agarose gel or nylon blot).

A nucleic acid molecule of the present invention, e.g., a nucleic acid molecule comprising a nucleotide sequence of SEQ ID NO:1, or a portion thereof, can be isolated using standard molecular biology techniques and the sequence information provided herein. For example, a *C*. *nicotianae* PDH cDNA that provides for a plant's increased resistance to a CBI compound can be isolated from a *C*. *nicotianae* library using all or portion of the sequence of SEQ ID NO:5. Moreover, a nucleic acid molecule encompassing all or a portion of SEQ ID NO: 1, can be isolated by polymerase chain reaction (PCR) using oligonucleotide primers designed based upon these sequences. For example, mRNA can be isolated from cells (e.g., by the guanidinium-thiocyanate extraction procedure of Chirgwin et al., 1979 Biochemistry 18:5294-5299) and cDNA can be prepared using reverse transcriptase (e.g., Moloney MLV reverse transcriptase, available from Gibco/BRL, Bethesda, MD; or AMV reverse transcriptase, available from Seikagaku America, Inc., St. Petersburg, FL). Synthetic oligonucleotide primers for PCR amplification can be designed based upon one of the nucleotide sequence shown in SEQ ID NO:1. A nucleic acid molecule of the invention can be amplified using cDNA or, alternatively, genomic DNA, as a template and appropriate oligonucleotide primers according to standard PCR amplification techniques. The nucleic acid molecule so amplified can be cloned into an appropriate vector and characterized by DNA sequence analysis. Furthermore, oligonucleotides corresponding to a CBIRP nucleotide sequence can be prepared by standard synthetic techniques, e.g., using an automated DNA synthesizer.

In a preferred embodiment, a nucleic acid molecule of the invention comprises the nucleotide sequence shown in SEQ ID NO:1 . These nucleotide sequences comprise only the coding region. Alternatively, the nucleic acid molecules of the present invention can include the coding region, as well as 5' untranslated sequences and 3' untranslated sequences of the sequence in SEQ ID NO:1, or can contain whole genomic fragments isolated from genomic DNA. Accordingly, the present invention includes CBIRP nucleic acids comprising the nucleotide sequence shown in SEQ ID NO:1 . The present invention also describes nucleic acids that encode CBIRPs as described herein. Preferred is a nucleic acid that encodes a CBIRP selected from the polypeptide sequence shown in SEQ ID NO:2 . Moreover, the nucleic acid molecule of the invention can comprise a portion of the coding region of the sequence in SEQ ID NO: 1, for example, a fragment which can be used as a probe or primer or a fragment encoding a biologically active portion of a CBIRP.

As used herein, the term "biologically active portion of" a CBIRP is intended to include a portion, e.g., a domain/motif of a CBIRP that catalyzes the conversion of phytoene to a colored carotenoid. More preferably, the biologically active portion of the CBIRP contributes to increased CBI resistance in a plant when expressed therein. For the purposes of the present invention, "increased" CBI resistance is determined as compared to a non-transgenic control plant. In a preferred embodiment, CBI resistance is increased by at least 2-fold in a transgenic plant comprising a CBIRP coding nucleic acid as compared to the CBI resistance of a non-transgenic control plant. In one embodiment, transgenic plants are about 4 cm tall, 10 cm in diameter, and at the four-leaf stage when the herbicide is sprayed. In a preferred embodiment, norflurazon is sprayed at concentrations of between about 10 and about 3,333 g/ha; fluridone is sprayed at a concentration of between about 10 and about 100 g/ha; or AC900001 is sprayed at a concentration between about of 10 and about 100 g/ha. Methods for quantitating CBI resistance are provided at least in Examples 5 below.

Biologically active portions of a CBIRP include peptides comprising amino acid sequences derived from the amino acid sequence of a CBIRP, e.g., an amino acid sequence of SEQ ID NO:2, or the amino acid sequence of a polypeptide identical to a CBIRP, which include fewer amino acids than a full length CBIRP or the full length polypeptide which is identical to a CBIRP, and exhibit at least one activity of a CBIRP. Typically, biologically active portions (e.g., peptides which are, for example, 5, 10, 15, 20, 30, 35, 36, 37, 38, 39, 40, 50, 100, or more amino acids in length) comprise a domain or motif with at least one activity of a CBIRP. Moreover, other biologically active portions in which other regions of the polypeptide are deleted, can be prepared by recombinant techniques and evaluated for one or more of the activities described herein.

The invention also provides CBIRP chimeric or fusion polypeptides. As used herein, a CBIRP "chimeric polypeptide" or "fusion polypeptide" comprises a CBIRP operably linked to a non-CBIRP. A CBIRP refers to a polypeptide having an amino acid sequence corresponding to a CBIRP, whereas a non-CBIRP refers to a polypeptide having an amino acid sequence corresponding to a polypeptide which is not substantially identical to the CBIRP, e.g., a polypeptide that is different from the CBIRP and is derived from the same or a different organism. Within the fusion polypeptide, the term "operably linked" is intended to indicate that the CBIRP and the non-CBIRP are fused to each other so that both sequences fulfill the proposed function attributed to the sequence used. The non-CBIRP can be fused to the N-terminus or C-terminus of the CBIRP. For example, in one embodiment, the fusion polypeptide is a GST-CBIRP fusion polypeptide in which the CBIRP sequences are fused to the C-terminus of the GST sequences. Such fusion polypeptides can facilitate the purification of recombinant CBIRPs. In another embodiment, the fusion polypeptide is a CBIRP containing a heterologous signal sequence at its N-terminus. In certain host cells (e.g., mammalian host cells), expression and/or secretion of a CBIRP can be increased through use of a heterologous signal sequence. Preferably, the chimeric or fusion polypeptide or the invention is produced by standard recombinant DNA techniques.

In addition to fragments and fusion polypeptides of the CBIRPs described herein, the present invention includes homologs and analogs of CBIRPs and CBIRP encoding nucleic acids in a plant. "Homologs" are defined herein as two nucleic acids or polypeptides that have similar, or "identical," nucleotide or amino acid sequences, respectively. Homologs include allelic variants, orthologs, paralogs, agonists, and antagonists of CBIRPs as defined hereafter. The term "homolog" further encompasses nucleic acid molecules that differ from the nucleotides sequence shown in SEQ ID NO:1, (and portions thereof) due to degeneracy of the genetic code, and thus encode the same CBIRP as that encoded by the nucleotide sequence shown in SEQ ID NO:1 .

To determine the percent sequence identity of two amino acid sequences (e.g., the sequence of SEQ ID NO:2, and a mutant form thereof), the sequences are aligned for optimal comparison purposes (e.g., gaps can be introduced in the sequence of one polypeptide for optimal alignment with the other polypeptide or nucleic acid). The amino acid residues at corresponding amino acid positions are then compared. When a position in one sequence (e.g., the sequence of SEQ ID NO:2, ) is occupied by the same amino acid residue as the corresponding position in the other sequence (e.g., a mutant form of the sequence of SEQ ID NO:2, SEQ ID NO:4, SEQ ID NO:6, or SEQ ID NO-8), then the molecules are identical at that position. The same type of comparison can be made between two nucleic acid sequences.

The percent sequence identity between the two sequences is a function of the number of identical positions shared by the sequences (i.e., percent sequence identity = numbers of identical positions/total numbers of positions x 100). Preferably, the isolated amino acid homologs included in the present invention are at least about 50-60%, preferably at least about 60-70%, and more preferably at least about 70-75%, 75-80%, 80-85%, 85-90% or 90-95%, and most preferably at least about 96%, 97%, 98%, 99% or more identical to an entire amino acid sequence shown in SEQ ID NO:2. In yet another embodiment, the isolated amino acid homologs included in the present invention are at least about 50-60%, preferably at least about 60-70%, and more preferably at least about 70-75%, 75-80%, 80-85%, 85-90% or 90-95%, and most preferably at least about 96%, 97%, 98%, 99% or more identical to an entire amino acid sequence encoded by a nucleic acid sequence, shown in SEQ ID NO:1. In other embodiments, the CBIRP homologs have sequence identity over at least 15 contiguous amino acid residues, more preferably at least 25 contiguous amino acid residues, and most preferably at least 35 contiguous amino acid residues of SEQ ID NO:2 .

In another preferred embodiment, an isolated nucleic acid homolog of the invention comprises a nucleotide sequence which is at least about 50-60%, preferably at least about 60-70%, more preferably at least about 70-75%, 75-80%, 80-85%, 85-90% or 90-95%, and even more preferably at least about 95%, 96%, 97%, 98%, 99% or more identical to a nucleotide sequence shown in SEQ ID NO: 1, or to a portion comprising at least 60 consecutive nucleotides thereof. The preferable length of sequence comparison for nucleic acids is at least 75 nucleotides, more preferably at least 100 nucleotides and most preferably the entire length of the coding region.

It is further preferred that the isolated nucleic acid homolog of the invention encodes a CBIRP, or portion thereof, that is at least 70% identical to an amino acid sequence of SEQ LID NO:2, and that catalyzes the conversion of phytoene to a colored carotenoid and that functions as a modulator of CBI resistance in a plant.

For the purposes of the invention, the percent sequence identity between two nucleic acid or polypeptide sequences is determined using the Vector NTI 6.0 (PC) software package (InforMax, 7600 Wisconsin Ave., Bethesda, MD 20814). A gap opening penalty of 15 and a gap extension penalty of 6.66 are used for determining the percent identity of two nucleic acids. A gap opening penalty of 10 and a gap extension penalty of 0.1, are used for determining the percent identity of two polypeptides. All other parameters are set at the default settings. It is to be understood that for the purposes of determining sequence identity when comparing a DNA sequence to an RNA sequence, a thymidine nucleotide is equivalent to a uracil nucleotide.

If another aspect, the invention provides an isolated nucleic acid comprising a polynucleotide that hybridizes to the polynucleotide of SEQ ID NO:1, under stringent conditions and that increases CBI resistance in a plant comprising the polynucleotide. More particularly, an isolated nucleic acid molecule of the invention is at least 15 nucleotides in length and hybridizes under stringent conditions to the nucleic acid molecule comprising a nucleotide sequence of SEQ ID NO:1, and that increases CBI resistance in a plant comprising the polynucleotide. In other embodiments, the nucleic acid is at least 30, 50, 100, 250 or more nucleotides in length. Preferably, an isolated nucleic acid homolog of the invention comprises a nucleotide sequence that hybridizes under highly stringent conditions to the nucleotide sequence shown in SEQ ID NO:1, and that increases CBI resistance in a plant comprising the polynucleotide.

As used herein with regard to hybridization for DNA to DNA blot, the term "stringent conditions" refers to hybridization overnight at 60°C in 10X Denhart's solution. 6X SSC, 0-5% SDS and 100 µg/ml denatured salmon sperm DNA. Blots are washed sequentially at 62°C for 30 minutes each time in 3X SSC/0.1% SDS, followed by 1X SSC/0.1% SDS, and finally 0.1X SSC/0.1% SDS. As also used herein, "highly stringent conditions" refers to hybridization overnight at 65°C in 10X Denhart's solution, 6X SSC, 0.5% SDS and 100 µg/ml denatured salmon sperm DNA. Blots are washed sequentially at 65°C for 30 minutes each time in 3X SSC/0.1% SDS, followed by 1X SSC/0.1% SDS, and finally 0.1X SSC/0.1% SDS. Methods for nucleic acid hybridizations are described in Meinkoth and Wahl, 1984 Anal. Biochem. 138:267-284; Current Protocols in Molecular Biology, Chapter 2, Ausubel et al. Eds., Greene Publishing and Wiley-Interscience, New York, 1995; and Tijssen, Laboratory Techniques in Biochemistry and Molecular Biology: Hybridization with Nucleic Acid Probes, Part I, Chapter 2, Elsevier, New York, 1993. '

Using the above-described methods, and others known to those of skill in the art, one of ordinary skill in the art can isolate homologs of nucleic acid molecules encoding the CBIRPs comprising an amino acid sequence shown in SEQ ID NO:2 . One subset of these homologs is allelic variants. As used herein, the term "allelic variant" refers to a nucleotide sequence containing polymorphisms that lead to changes in the amino acid sequences of a CBIRP and that exist within a natural population (e.g., a plant species or variety). Such natural allelic variations can typically result in 1-5% variance in a CHIRP nucleic acid. Allelic variants can be identified by sequencing the nucleic acid sequence of interest in a number of different plants, which can be readily carried out by using hybridization probes to identify the same CBIRP genetic locus in those plants. Any and; all such nucleic acid variations and resulting amino acid polymorphisms or variations in a CBIRP that are the result of natural allelic variation, and that do not alter the functional activity of a CBIRP, are intended to be within the scope of the invention.

Moreover, nucleic acid molecules encoding CBIRPs from the same or other species such as analogs, orthologs and paralogs, are intended to be within the scope of the present invention. As used herein, the term "analogs" refers to two nucleic acids that have the same or similar function, but that have evolved separately in unrelated organisms. As used herein, the term "orthologs" refers to two nucleic acids from different species, but that have evolved from a common ancestral gene by speciation. Normally, orthologs encode polypeptides having the same or similar functions. As also used herein, the term "paralogs" refers to two nucleic acids that are related by duplication within a genome. Paralogs usually have different functions, but these functions may be related (Tatusov, R.L. et al., 1997 Science 278(5338):631-637). Analogs, orthologs and paralogs of a CBIRP can differ from the CBIRP by post-translational modifications, by amino acid sequence differences, or by both. Post-translational modifications include *in vivo* and *in vitro* chemical derivatization of polypeptides, e.g., acetylation, carboxylation, phosphorylation, or glycosylation, and such modifications may occur during polypeptide synthesis or processing or following treatment with isolated modifying enzymes. In particular, orthologs of the invention will generally exhibit at least 80-85%, more preferably, 85-90% or 90-95%, and most preferably 95%, 96%, 97%, 98% or even 99% identity or sequence identity with all or part of a CBIRP amino acid sequence and will exhibit a function similar to the CBIRP. Preferably, a CBIRP ortholog of the present invention catalyzes the conversion of phytoene to a colored carotenoid and functions as a contributor to increased CBI resistance in a plant, and more preferably, increases CBI resistance in a plant when expressed therein.

In addition to naturally-occurring variants of a CBIRP sequence that may exist in the population, the skilled artisan will further appreciate that changes can be introduced by mutation into a nucleotide sequence of SEQ ID NO:1, thereby leading to changes in the amino acid sequence of the encoded CBIRP, without altering the functional activity of the CBIRP. For example, nucleotide substitutions leading to amino acid substitutions at "non-essential" amino acid residues can be made in a sequence of SEQ ID NO:1. A "non-essential" amino acid residue is a residue that can be altered from the sequence of one of the CBIRPs without altering the activity of said CBIRP, whereas an "essential" amino acid residue is required for increased CBI resistance. Accordingly, another aspect of the invention pertains to nucleic acid molecules encoding CBIRPs that contain changes in amino acid residues that are not essential for increased CBI resistance.

Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a predicted nonessential amino acid residue in a CBIRP is preferably replaced with another amino acid residue from the same side chain family. Alternatively, in another embodiment, mutations can be introduced randomly along all or part of a CBIRP coding sequence, such as by saturation mutagenesis, and the resultant mutants can be screened for a CBIRP activity described herein to identify mutants that retain CBIRP activity. Following mutagenesis of the sequence of SEQ ID NO:1, the encoded polypeptide can be expressed recombinantly and the activity of the polypeptide can be determined by analyzing the CBI resistance of a plant expressing the polypeptide as described in Example 6.

Additionally, optimized CBIRP coding nucleic acids can be created. Preferably, an optimized CBIRP nucleic acid encodes a CBIRP that binds to DNA, functions as a transcription factor, and/or modulates a plant's tolerance to an environmental stress, and more preferably increases a plant's tolerance to an environmental stress upon its over-expression in the plant. As used herein, "optimized" refers to a nucleic acid that is genetically engineered to increase its expression in a given plant or animal. To provide plant optimized CBIRP nucleic acids, the DNA sequence of the gene can be modified to 1) comprise codons preferred by highly expressed plant genes; 2) comprise an A+T content in nucleotide base composition to that substantially found in plants; 3) form a plant initiation sequence, 4) to eliminate sequences that cause destabilization, inappropriate polyadenylation, degradation and termination of RNA, or that form secondary structure hairpins or RNA splice sites. Increased expression of CBIRP nucleic acids in plants can be achieved by utilizing the distribution frequency of codon usage in plants in general or a particular plant. Methods for optimizing nucleic acid expressions plants can be found in EPA 0359472; EPA 0385962; PCT Application No. WO 91/16432; U.S. Patent No. 5,380,831; U.S. Patent No. 5,436,391; Perlack et al., 1991 Proc. Natl. Acad. Sci. USA 88:3324-3328; and Murray et al. 1989 Nucleic Acids Res. 17:477-498.

As used herein, "frequency of preferred codon usage" refers to the reference exhibited by a specific host cell in usage of nucleotide codons to specify a given amino acid. To determine the frequency of usage of a particular codon in a gene, the number of occurrences of that codon in the gene is divided by the total number of occurrences of all codons specifying the same amino acid in the gene. Similarly, the frequency of preferred codon usage exhibited by a host cell can be calculated by averaging frequency of preferred codon usage in a large number of genes expressed by the host cell. It is preferable that this analysis be limited to genes that are highly expressed by the host cell. The percent deviation of the frequency of preferred codon usage for a synthetic gene from that employed by a host cell is calculated first by determining the percent deviation of the frequency of usage of a single codon from that of the host cell followed by obtaining the average deviation over all codons. As defined herein, this calculation includes unique codons (i.e., ATG and TGG). In general terms, the overall average deviation of the codon usage of an optimized gene from that of a host cell is calculated using the equation 1A = n = 1 Z Xₙ - Yₙ Xₙ times 100 Z where Xₙ = frequency of usage for codon n in the host cell; Yₙ = frequency of usage for codon n in the synthetic gene, n represents an individual codon that specifies an amino acid and the total number of codons is Z. The overall deviation of the frequency of codon usage, A, for all amino acids should preferably be less than about 25%, and more preferably less than about 10%.

Hence, a CBIRP coding nucleic acid can be optimized such that its distribution frequency of codon usage deviates, preferably, no more than 25% from that of highly expressed plant genes and, more preferably, no more than about 10%. In addition, consideration is given to the percentage G+C content of the degenerate third base (monocotyledons appear to favor G+C in this position, whereas dicotyledons do not). It is also recognized that the XCG (where X is A, T, C, or G) codon is the least preferred codon in dicots whereas the XTA codon is avoided in both monocots and dicots. Optimized CBIRP coding nucleic acids of this invention also preferably have CG and TA doublet avoidance indices closely approximating those of the chosen host plant. More preferably these indices deviate from that of the host by no more than about 10-15%.

In addition to the CBIRP nucleic acids and polypeptides described above, the present invention encompasses these nucleic acids and polypeptides attached to a moiety. These moieties include, but are not limited to, detection moieties, hybridization moieties, purification moieties, delivery moieties, reaction moieties, binding moieties, and the like. A typical group of nucleic acids having moieties attached are probes and primers. Probes and primers typically comprise a substantially isolated oligonucleotide. The oligonucleotide typically comprises a region of nucleotide sequence that hybridizes under stringent conditions to at least about 12, preferably about 25, more preferably about 40, 50 or 75 consecutive nucleotides of a sense strand of one of the sequences set forth in SEQ ID NO:1, or naturally occurring mutants thereof. Probes based on the CBIRP nucleotide sequences can be used to detect transcripts or genomic sequences encoding the same or substantially identical polypeptides. In preferred embodiments, the probe further comprises a label group attached thereto, e.g. the label group can be a radioisotope, a fluorescent compound, an enzyme, or an enzyme co-factor. Such probes can be used as a part of a genomic marker test kit for identifying cells which express a CBIRP, such as by measuring a level of a CBIRP-encoding nucleic acid, in a sample of cells, e.g., detecting CHIRP mRNA levels or determining whether a genomic CBIRP gene has been mutated or deleted.

The invention further provides an isolated recombinant expression vector comprising a CBIRP nucleic acid as described above, wherein expression of the CBIRP nucleic acid in a host cell results in increased resistance to a CBI compound as compared to a wild type variety of the host cell. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of polynucleotides to which they are operably linked. Such vectors are referred to herein as "expression vectors-" In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (e.g., replication defective retroviruses, adenoviruses and adeno-associated viruses), which serve equivalent functions. As used herein, the term "nuclear expression vector" refers to a vector that targets the nucleus of the host cell for expression of a nucleic acid contained in the vector. The term "plastid expression vector" refers to a vector that targets a plastid, such as a chloroplast, for the expression of a nucleic acid contained in the vector.

The recombinant expression vectors of the invention comprise a CBIRP coding nucleic acid of the invention in a form suitable for expression of the nucleic acid in a host cell, which means that the recombinant expression vectors include one or more regulatory sequences, selected on the basis of the host cells to be used for expression, which is operably linked to the nucleic acid sequence to be expressed. Within a recombinant expression vector, "operably linked" is intended to mean that the nucleotide sequence of interest is linked to the regulatory sequence(s) in a manner which allows for expression of the nucleotide sequence (e.g., in an *in vitro* transcription/translation system or in a host cell when the vector is introduced into the host cell). The term "regulatory sequence" is intended to include promoters, enhancers and other expression control elements (e.g., polyadenylation signals). Such regulatory sequences are described, for example, in Goeddel, Gene Expression Technology: Methods in Enzymology 185, Academic Press, San Diego, CA (1990) or see: Gruber and Crosby, in: Methods in Plant Molecular Biology and Biotechnology, eds. Glick and Thompson, Chapter 7, 89-108, CRC Press: Boca Raton, Florida, including the references therein. Regulatory sequences include those that direct constitutive expression of a nucleotide sequence in many types of host cells and those that direct expression of the nucleotide sequence only in certain host cells or under certain conditions. It will be appreciated by those skilled in the art that the design of the expression vector can depend on such factors as the choice of the host cell to be transformed, the level of expression of polypeptide desired, etc. The expression vectors of the invention can be introduced into host cells to thereby produce polypeptides or peptides, including fusion polypeptides or peptides, encoded by nucleic acids as described herein (e.g., CBIRPs, fusion polypeptides, etc.).

The recombinant expression vectors of the invention can be designed for expression of CBIRPs in prokaryotic or eukaryotic cells. For example, CBIRP genes can be expressed in bacterial cells such as *C. glutamicum,* insect cells (using baculovirus expression vectors), yeast and other fungal cells (see Romanos, M.A. et al., 1992 Foreign gene expression in yeast: a review, Yeast 8:423-488; van den Hondel, C.A.M.J.J. et al., 1991 Heterologous gene expression in filamentous fungi, in: More Gene Manipulations in Fungi, J.W. Bennet & L.L. Lasure, eds., p. 396-428: Academic Press: San Diego; and van den Hondel, C.A.M.J.J. & Punt, P.J., 1991 Gene transfer systems and vector development for filamentous fungi, in: Applied Molecular Genetics of Fungi, Peberdy, J.F. et al., eds., p. 1-28, Cambridge University Press: Cambridge), algae (Falciatore et al., 1999 Marine Biotechnology 1(3):239-251), ciliates of the types: Holotrichia, Peritrichia, Spirotrichia, Suctoria, Tetrahymena, Paramecium, Colpidium, Glaucoma, Platyophrya, Potomacus, Pseudocohnilembus, Euplotes, Engelmaniella, and Stylonychia, especially of the genus Stylonychia lemnae with vectors following a transformation method as described in PCT Application No. WO 98/01572 and multicellular plant cells (see Schmidt, R. and Willmitzer, L., 1988 High efficiency Agrobacterium tumefaciens-mediated transformation of Arabidopsis thaliana leaf and cotyledon explants, Plant Cell Rep. 583-586; Plant Molecular Biology and Biotechnology, C Press, Boca Raton, Florida, chapter 6/7, S.71-119 (1993); F.F. White, B. Jenes et al., Techniques for Gene Transfer, in: Transgenic Plants, Vol. 1, Engineering and Utilization, eds. Kung and R. Wu, 128-43, Academic Press: 1993; Potrykus, 1991 Annu. Rev. Plant Physiol. Plant Molec. Biol. 42:205-225 and references cited therein) or mammalian cells. Suitable host cells are discussed further in Goeddel, Gene Expression Technology: Methods in Enzymology 185, Academic Press: San Diego, CA (1990). Alternatively, the recombinant expression vector can be transcribed and translated *in vitro,* for example using T7 promoter regulatory sequences and T7 polymerase.

Expression of polypeptides in prokaryotes is most often carried out with vectors containing constitutive or inducible promoters directing the expression of either fusion or non-fusion polypeptides. Fusion vectors add a number of amino acids to a polypeptide encoded therein, usually to the amino terminus of the recombinant polypeptide but also to the C-terminus or fused within suitable regions in the polypeptides. Such fusion vectors typically serve three purposes: 1) to increase expression of a recombinant polypeptide; 2) to increase the solubility of a recombinant polypeptide; and 3) to aid in the purification of a recombinant polypeptide by acting as a ligand in affinity purification. Often, in fusion expression vectors, a proteolytic cleavage site is introduced at the junction of the fusion moiety and the recombinant polypeptide to enable separation of the recombinant polypeptide from the fusion moiety subsequent to purification of the fusion polypeptide. Such enzymes, and their cognate recognition sequences, include Factor Xa, thrombin and enterokinase.

Typical fusion expression vectors include pGEX (Pharmacia Biotech Inc; Smith, D.B. and Johnson, K.S., 1988 Gene 67:31-40), pMAL (New England Biolabs, Beverly, MA) and pRIT5 (Pharmacia, Piscataway, NJ) which fuse glutathione S-transferase (GST), maltose E binding polypeptide, or polypeptide A, respectively, to the target recombinant polypeptide. In one embodiment, the coding sequence of the CBIRP is cloned into a pGEX expression vector to create a vector encoding a fusion polypeptide comprising, from the N-terminus to the C-terminus, GST-thrombin cleavage site-X polypeptide. The fusion polypeptide can be purified by affinity chromatography using glutathione-agarose resin. Recombinant CBIRP unfused to GST can be recovered by cleavage of the fusion polypeptide with thrombin.

Examples of suitable inducible non-fusion *E. coli* expression vectors include pTrc (Amann et al., 1988 Gene 69:301-315) and pET 11d (Studier et al., Gene Expression Technology: Methods in Enzymology 185, Academic Press, San Diego, California (1990) 60-89). Target gene expression from the pTrc vector relies on host RNA polymerase transcription from a hybrid trp-lac fusion promoter. Target gene expression from the pET 11d vector relies on transcription from a T7 gn10-lac fusion promoter mediated by a co-expressed viral RNA polymerase (T7 gnl). This viral polymerase is supplied by host strains BL21(DE3) or HMS174(DE3) from a resident λ prophage harboring a T7 gnl gene under the transcriptional control of the lacUV 5 promoter.

One strategy to maximize recombinant polypeptide expression is to express the polypeptide in a host bacteria with an impaired capacity to proteolytically cleave the recombinant polypeptide (Gottesman, S., Gene Expression Technology: Methods in Enzymology 185, Academic Press, San Diego, California (1990) 119-128). Another strategy is to alter the sequence of the nucleic acid to be inserted into an expression vector so that the individual codons for each amino acid are those preferentially utilized in the bacterium chosen for expression, such as *C. glutamicum* (Wada et al., 1992 Nucleic Acids Res. 20:2111-2118). Such alteration of nucleic acid sequences of the invention can be carried out by standard DNA synthesis techniques.

In another embodiment, the CBIRP expression vector is a yeast expression vector. Examples of vectors for expression in yeast *S. cerevisiae* include pYepSec 1 (Baldari et al., 1987 EMBO J. 6:229-234), pMFa (Kurjan and Herskowitz, 1982 Cell 30:933-943), pJRY88 (Schultz et al., 1987 Gene 54:113-123), and pYES2 (Invitrogen Corporation, San Diego, CA). Vectors and methods for the construction of vectors appropriate for use in other fungi, such as the filamentous fungi, include those detailed in: van den Hondel, C.A.M.J.J. & Punt, P.J. (1991) "Gene transfer systems and vector development for filamentous fungi," in: Applied Molecular Genetics of Fungi, J.F. Peberdy, et al., eds., p. 1-28, Cambridge University Press: Cambridge.

In a preferred embodiment of the present invention, the CBIRPs are expressed in plants and plants cells such as unicellular plant cells (such as algae) (see Falciatore et al., 1999 Marine Biotechnology 1(3):239-251 and references therein) and plant cells from higher plants (e.g., the spermatophytes, such as crop plants). A CBIRP may be "introduced" into a plant cell by any means, including transfection, transformation or transduction, electroporation, particle bombardment, agroinfection and the like. One transformation method known to those of skill in the art is the dipping of a flowering plant into an Agrobacteria solution, wherein the Agrobacteria contain the CBIRP nucleic acid, followed by breeding of the transformed gametes.

Other suitable methods for transforming or transfecting host cells including plant cells can be found in Sambrook, et al. (Molecular Cloning: A Laboratory Manual. 2nd, ed., Cold Spring Harbor Laboratory, Cold Spring Harbor Laboratory Press, Cold Spring Harbor, NY, 1989) and other laboratory manuals such as Methods in Molecular Biology, 1995, Vol. 44, Agrobacterium protocols, ed: Gartland and Davey, Humana Press, Totowa, New Jersey. As increased CBI resistance is a general trait wished to be inherited into a wide variety of plants like maize, wheat, rye, oat, triticale, rice, barley, soybean, peanut, cotton, rapeseed and canola, manihot, pepper, sunflower and tagetes, solanaceous plants like potato, tobacco, eggplant, and tomato, Vicia species, pea, alfalfa, bushy plants (coffee, cacao, tea), Salix species, trees (oil palm, coconut), perennial grasses and forage crops, these crop plants are also preferred target plants for a genetic engineering as one further embodiment of the present invention. Forage crops include, but are not limited to, Wheatgrass, Canarygrass, Bromegrass, Wildrye Grass, Bluegrass, Orchardgrass, Alfalfa, Salfoin, Birdsfoot Trefoil, Alsike Clover, Red Clover and Sweet Clover.

In one embodiment of the present invention, transfection of a CBIRP into a plant is achieved by *Agrobacterium* mediated gene transfer. *Agrobacterium* mediated plant transformation can be performed using for example the GV3101(pMP90) (Koncz and Schell, 1986 Mol. Gen. Genet. 204:383-396) or LBA4404 (Clontech) *Agrobacterium tumefaciens* strain. Transformation can be performed by standard transformation and regeneration techniques (Deblaere et al., 1994 Nucl. Acids. Res. 13:4777-4788; Gelvin, Stanton B. and Schilperoort, Robert A, Plant Molecular Biology Manual, 2nd Ed. - Dordrecht: Kluwer Academic Publ., 1995. - in Sect., Ringbuc Zentrale Signatur: BT11-P ISBN 0-7923-2731-4; Glick, Bernard R.; Thompson, John E., Methods in Plant Molecular Biology and Biotechnology, Boca Raton : CRC Press, 1993 360 S., ISBN 0-8493-5164-2). For example, rapeseed can be transformed via cotyledon or hypocotyl transformation (Moloney et al., 1989 Plant cell Report 8:238-242; De Block et al., 1989 Plant Physiol. 91:694-701). Use of antibiotics for *Agrobacterium* and plant selection depends on the binary vector and the *Agrobacterium* strain used for transformation. Rapeseed selection is normally performed using gentamycin as selectable plant marker. *Agrobacterium* mediated gene transfer to flax can be performed using, for example, a technique described by Mlynarova et al., 1994 Plant Cell Report 13:282-285. Additionally, transformation of soybean can be performed using for example a technique described in European Patent No. 0424 047, U.S. Patent No. 5,322,783, European Patent No. 0397 687, U.S. Patent No. 5,376,543 or U.S. Patent No. 5,169,770. Transformation of maize can be achieved by particle bombardment, polyethylene glycol mediated DNA uptake, or the silicon carbide fiber technique. (See, for example, Freeling and Walbot "The maize handbook" Springer Verlag: New York (1993) ISBN 3-540-97826-7). A specific example of maize transformation is found in U.S. Patent No. 5,990,387 and a specific example of wheat transformation can be found in PCT Application No. WO 93/07256.

In another embodiment of the present invention, particle bombardment is used to introduce the CBIRP coding nucleic acid into the plant. Particle bombardment can be performed by standard techniques as described in Klein et al., 1987, Nature, 327:70-73.

According to the present invention, the introduced CBIRP may be maintained in the plant cell stably if it is incorporated into a non-chromosomal autonomous replicon or integrated into the plant or chloroplast genomes. Alternatively, the introduced CBIRP may be present on an extra-chromosomal non-replicating vector and be transiently expressed or transiently active.

In one embodiment, a homologous recombinant microorganism can be created wherein the CBIRP is integrated into a chromosome. A vector is prepared which contains the gene encoding the CBIRP. Preferably, the CBIRP is from *Arabidopsis thaliana, Synechocystis* PCC6803, *Cercospora nicotianae*, barley, wheat, corn, canola, or soybean. More preferably, the CBIRP is from barley, wheat, corn, canola, or soybean. To create a point mutation via homologous recombination, DNA-RNA hybrids can be used in a technique known as chimeraplasty (Cole-Strauss et al., 1999 Nucleic Acids Research 27(5):1323-1330 and Kmiec, 1999 Gene therapy American Scientist 87(3):240-247). Homologous recombination procedures in these species are also well known in the art and are contemplated for use herein.

In the homologous recombination vector, the altered portion of the CBIRP gene is flanked at its 5' and 3' ends by an additional nucleic acid molecule of the CBIRP gene to allow for homologous recombination to occur between the exogenous CBIRP gene carried by the vector and an endogenous CBIRP gene, in a microorganism or plant. Alternatively, the CBIRP nucleic acid could be flanked in the vector with polynucleotide sequences native to the target plant that are not essential for the efficient growth and maintenance of that target plant, allowing the CBIRP nucleic acid to replace that polynucleotide sequence upon homologous recombination. The additional flanking CBIRP nucleic acid or other polynucleotide sequence is of sufficient length for successful homologous recombination with the endogenous gene. Typically, several hundreds of base pairs up to kilobases of flanking DNA (both at the 5' and 3' ends) are included in the vector (see e.g., Thomas, K.R., and Capecchi, M.R., 1987 Cell 51:503 for a description of homologous recombination vectors or Strepp et al., 1998 PNAS, 95 (8):4368-4373 for cDNA based recombination in *Physcomitrella patens*). The vector is introduced into a microorganism or plant cell (e.g., via polyethylene glycol mediated DNA), and cells in which the introduced CBIRP gene has homologously recombined with the endogenous CBIRP gene are selected using techniques known in the art.

Whether present in an extra-chromosomal non-replicating vector or a vector that is integrated into a chromosome, the CBIRP coding nucleic acid preferably resides in a plant expression cassette. A plant expression cassette preferably contains regulatory sequences capable of driving gene expression in plant cells that are operably linked so that each sequence can fulfill its function, for example, termination of transcription by polyadenylation signals. Preferred polyadenylation signals are those originating from *Agrobacterium tumefaciens* t-DNA such as the gene 3 known as octopine synthase of the Ti-plasmid pTiACH5 (Gielen et al., 1984 EMBO J. 3:835) or functional equivalents thereof but also all other terminators functionally active in plants are suitable. As plant gene expression is very often not limited on transcriptional levels, a plant expression cassette preferably contains other operably linked sequences like translational enhancers such as the overdrive-sequence containing the 5'-untranslated leader sequence from tobacco mosaic virus enhancing the polypeptide per RNA ratio (Gallie et al., 1987 Nucl. Acids Research 15:8693-8711). Examples of plant expression vectors include those detailed in: Becker, D., Kemper, E., Schell, J. and Masterson, R., 1992 New plant binary vectors with selectable markers located proximal to the left border, Plant Mol. Biol. 20:1195-1197; and Bevan, M.W., 1984 Binary Agrobacterium vectors for plant transformation, Nucl. Acid. Res. 12:8711-8721; Vectors for Gene Transfer in Higher Plants; in: Transgenic Plants, Vol. 1, Engineering and Utilization, eds.: Kung and R. Wu, Academic Press, 1993, S. 15-38.

Plant gene expression should be operably linked to an appropriate promoter conferring gene expression in a timely, cell or tissue specific manner. Promoters useful in the expression cassettes of the invention include any promoter that is capable of initiating transcription in a plant cell. Such promoters include, but are not limited to those that can be obtained from plants, plant viruses and bacteria that contain genes that are expressed in plants, such as *Agrobacterium* and *Rhizobium*.

The promoter may be constitutive, inducible, developmental stage-preferred, cell type-preferred, tissue-preferred or organ-preferred. Constitutive promoters are active under most conditions. Examples of constitutive promoters include the CaMV 19S and 35 S promoters (Odell et al. 1985 Nature 313:810-812), the sX CaMV 35S promoter (Kay et al. 1987 Science 236:1299-1302) the Sepl promoter, the rice actin promoter (McElroy et al. 1990 Plant Cell 2:163-171), the *Arabidopsis* actin promoter, the ubiquitan promoter (Christensen et al. 1989 Plant Molec. Biol. 18:675-689); pEmu (Last et al. 1991 Theor Appl Genet 81:581-588), the figwort mosaic virus 35S promoter, the Smas promoter (Velten et al. 1984 EMBO J 3:2723-2730), the GRP1-8 promoter, the cinnamyl alcohol dehydrogenase promoter (U.S. Patent No. 5,683,439), promoters from the T-DNA of Agrobacterium, such as mannopine synthase, nopaline synthase, and octopine synthase, the small subunit of ribulose biphosphate carboxylase (ssuRUBISCO) prompter, and the like. In a preferred embodiment, the CBIRP nucleic acid is operably linked to a constitutive or tissue-preferred promoter. In a further preferred embodiment, the tissue-preferred promoter is a leaf-preferred promoter.

Inducible promoters are active under certain environmental conditions, such as the presence or absence of a nutrient or metabolite, heat or cold, light, pathogen attack, anaerobic conditions, and the like. For example, the hsp80 promoter from *Brassica* is induced by heat shock, the PPDK promoter is induced by light, the PR-1 promoter from tobacco, *Arabidopsis* and maize are inducible by infection with a pathogen, and the Adh1 promoter is induced by hypoxia and cold stress. Plant gene expression can also be facilitated via an inducible promoter (for review see Gatz, 1997 Annu. Rev. Plant Physiol. Plant Mol. Biol. 48:89-108). Chemically inducible promoters are especially suitable if gene expression is wanted to occur in a time specific manner. Examples of such promoters are a salicylic acid inducible promoter (PCT Application No. WO 95/19443), a tetracycline inducible promoter (Gatz et al., 1992 Plant J. 2:397-404) and an ethanol inducible promoter (PCT Application No. WO 93/21334). In one embodiment of the present invention, the inducible promoter is a stress-inducible promoter.

Developmental stage-preferred promoters are preferentially expressed at certain stages of development. Tissue and organ preferred promoters include those that are preferentially expressed in certain tissues or organs, such as leaves, roots, seeds, or xylem. Examples of tissue preferred and organ preferred promoters include, but are not limited to fruit-preferred, ovule-preferred, male tissue-preferred, seed-preferred, integument-preferred, tuber-preferred, stalk-preferred, pericarp-preferred, and leaf-preferred, stigma-preferred, pollen-preferred, anther-preferred, a petal-preferred, sepal-preferred, pedicel-preferred, silique-preferred, stem-preferred, root-preferred promoters and the like. Seed preferred promoters are preferentially expressed during seed development and/or germination. For example, seed preferred promoters can be embryo-preferred, endosperm preferred and seed coat-preferred. See Thompson et al. 1989 BioEssays 10:108. Examples of seed preferred promoters include, but are not limited to cellulose synthase (celA), Ciml, gamma-zein, globulin-1, maize 19 kD zein (cZ19B1) and the like.

Other suitable tissue-preferred or organ-preferred promoters include the napin-gene promoter from rapeseed (U.S. Patent No. 5,608,152), the USP-promoter from Vicia faba (Baeumlein et al., 1991 Mol Gen Genet. 225(3):459-67), the oleosin-promoter from *Arabidopsis* (PCT Application No. WO 98/45461), the phaseolin-promoter from *Phaseolus vulgaris* (U.S. Patent No. 5,504,200), the Bce4-promoter from *Brassica* (PCT Application No. WO 91/13980) or the legumin B4 promoter (LeB4; Baeumlein et al., 1992 Plant Journal, 2(2):233-9) as well as promoters conferring seed specific expression in monocot plants like maize, barley, wheat, rye, rice, etc. Suitable promoters to note are the lpt2 or lptl-gene promoter from barley (PCT Application No. WO 95/15389 and PCT Application No. WO 95/23230) or those described in PCT Application No. WO 99/16890 (promoters from the barley hordein-gene, rice glutelin gene, rice oryzin gene, rice prolamin gene, wheat gliadin gene, wheat glutelin gene, oat glutelin gene, Sorghum kasirin-gene and rye secalin gene).

Other promoters useful in the expression cassettes of the invention include, but are not limited to, the major chlorophyll a/b binding protein promoter, histone promoters, the Ap3 promoter, the β-conglycin promoter, the napin promoter, the soy bean lectin promoter, the maize 15kD zein promoter, the 22kD zein promoter, the 27kD zein promoter, the g-zein promoter, the waxy, shrunken 1, shrunken 2 and bronze promoters, the Zm13 promoter (U.S. Patent No. 5,086,169), the maize polygalacturonase promoters (PG) (U.S. Patent Nos. 5,412,085 and 5,545,546) and the SGB6 promoter (U.S. Patent No. 5,470,359), as well as synthetic or other natural promoters.

Additional flexibility in controlling heterologous gene expression in plants may be obtained by using DNA binding domains and response elements from heterologous sources (i.e., DNA binding domains from non-plant sources). An example of such a heterologous DNA binding domain is the LexA DNA binding domain (Brent and Ptashne, 1985 Cell 43:729-736).

Another aspect of the invention pertains to host cells into which a recombinant expression vector of the invention has been introduced. The terms "host cell" and "recombinant host cell" are used interchangeably herein. It is understood that such terms refer not only to the particular subject cell but they also apply to the progeny or potential progeny of such a cell. Because certain modifications may occur in succeeding generations due to either mutation or environmental influences, such progeny may not, in fact, be identical to the parent cell, but are still included within the scope of the term as used herein. A host cell can be any prokaryotic or eukaryotic cell. For example, a CBIRP can be expressed in bacterial cells such as C. *glutamicum,* insect cells, fungal cells or mammalian cells (such as Chinese hamster ovary cells (CHO) or COS cells), algae, ciliates, plant cells, fungi or other microorganisms like C. *glutamicum.* Other suitable host cells are known to those skilled in the art.

A host cell of the invention, such as a prokaryotic or eukaryotic host cell in culture, can be used to produce (i.e., express) a CBIRP. Accordingly, the invention further provides methods for producing CBIRPs using the host cells of the invention. In one embodiment, the method comprises culturing the host cell of invention (into which a recombinant expression vector encoding a CBIRP has been introduced, or into which genome has been introduced a gene encoding a CBIRP) in a suitable medium until CBIRP is produced. In another embodiment, the method further comprises isolating CBIRPs from the medium or the host cell.

Another aspect of the invention pertains to isolated CBIRPs, and biologically active portions thereof. An "isolated" or "purified" polypeptide or biologically active portion thereof is free of some of the cellular material when produced by recombinant DNA techniques, or chemical precursors or other chemicals when chemically synthesized. The language "substantially free of cellular material" includes preparations of CBIRP in which the polypeptide is separated from some of the cellular components of the cells in which it is naturally or recombinantly produced. In one embodiment, the language "substantially free of cellular material" includes preparations of a CBIRP having less than about 30% (by dry weight) of non-CBIRP material (also referred to herein as a "contaminating polypeptide"), more preferably less than about 20% of non-CBIRP material, still more preferably less than about 10% of non-CBIRP material, and most preferably less than about 5% non-CBIRP material.

When the CBIRP or biologically active portion thereof is recombinantly produced, it is also preferably substantially free of culture medium, i.e., culture medium represents less than about 20%, more preferably less than about 10%, and most preferably less than about 5% of the volume of the polypeptide preparation. The language "substantially free of chemical precursors or other chemicals" includes preparations of CBIRP in which the polypeptide is separated from chemical precursors or other chemicals that are involved in the synthesis of the polypeptide. In one embodiment, the language ""substantially free of chemical precursors or other chemicals" includes preparations of a CBIRP having less than about 30% (by dry weight) of chemical precursors or non-CBIRP chemicals, more preferably less than about 20% chemical precursors or non-CBIRP chemicals, still more preferably less than about 10% chemical precursors or non-CBIRP chemicals, and most preferably less than about 5% chemical precursors or non-CBIRP chemicals. In preferred embodiments, isolated polypeptides, or biologically active portions thereof, lack contaminating polypeptides from the same organism from which the CBIRP is derived. Typically, such polypeptides are produced by recombinant expression of a CHIRP in plants other than the plant from which the CBIRP was isolated or in microorganisms such as *C. glutamicum,* ciliates, algae or fungi.

The nucleic acid molecules, polypeptides, polypeptide homologs, fusion polypeptides, primers, vectors, and host cells described herein can be used in one or more of the following methods: determination of CBIRP regions required for function, modulation of a CBIRP activity; modulation of CBI resistance; and modulation of expression of CBIRP nucleic acids. More particularly, the nucleic acid and amino acid sequences of the present invention can be used to transform plants, thereby increasing resistance to a CBI compound, such as norflurazon, fluridone, AC 375488, and AC 900001. The present invention therefore provides a transgenic plant containing a CBIRP coding nucleic acid, wherein expression of the nucleic acid in the plant contributes to the plant's increased resistance to a CBI compound as compared to a wild type variety of the plant. The transgenic plant can be a monocot or a dicot. The invention further provides that the transgenic plant can be selected from maize, wheat, rye, oat, triticale, rice, barley, soybean, peanut, cotton, rapeseed, canola, manihot, pepper, sunflower, tagetes, solanaceous plants, potato, tobacco, eggplant, tomato, Vicia species, pea, alfalfa, coffee, cacao, tea, Salix species, oil palm, coconut, perennial grass and forage crops, for example.

In particular, the present invention describes using the expression of a CBI resistant PDS polynucleotide or *Arabidopsis thaliana* or *Synechocystis* PCC6803, or a CBI resistant PDH polynucleotide of *Cercospora nicotianae* to engineer plants with increased resistance to a CBI compound. This strategy has herein been demonstrated for tobacco, but its application is not restricted to this plant.

Accordingly, the invention provides that the transgenic plant comprises a CBIRP coding nucleic acid. In one embodiment of the present invention, the transgenic plant comprises an altered PDS nucleic acid. In a preferred embodiment, the altered PDS nucleic acid encodes an altered PDS protein from *Arabidopsis thaliana* comprising an arginine to proline, amino acid substitution at a position corresponding to position 288 in a wild type PDS protein from *Arabidopsis thaliana*. It is envisaged that, the altered PDS nucleic acid encodes an altered PDS protein from *Synechocystis* PCC6803 comprising an alanine to threonine amino acid substitution at a position corresponding to position 175 in a wild type PDS protein from *Synechocystis* PCC6803. It is described that the transgenic plant contains a PDS nucleic acid and the PDH nucleic acid is from *Cercospora nicotianae*. In a more preferred embodiment, the invention provides a transgenic plant containing a CBIRP coding nucleic acid selected from an altered PDS polynucleotide of *Arabidopsis thaliana* as defined in SEQ ID NO:1, wherein the plant has an increased resistance to a CBI compound.

Accordingly, the invention provides a method of producing a transgenic plant with a CBIRP coding nucleic acid, wherein expression of the nucleic acid(s) in the plant contributes to an increased resistance to a CBI compound as compared to a wild type variety of the plant comprising: (a) introducing into a plant cell a nuclear or plastid expression vector comprising a CBIRP nucleic acid, and (b) generating from the plant cell a transgenic plant with an increased resistance to a CBI compound as compared to a wild type variety of the plant, The plant cell includes, but is not limited to, a protoplast, gamete producing cell, and a cell that regenerates into a whole plant. As used herein, the term "Transgenic" refers to any plant, plant cell, callus, plant tissue or plant part, that contains all or part of at least one recombinant polynucleotide. The term "transgenic" includes a plant, plant cell, callus, plant tissue or plant part, wherein a recombinant polynucleotide is introduced into the nucleus or a plastid of a plant cell. In many cases, all or part of the recombinant polynucleotide is stably integrated into a chromosome or stable extra-chromosomal element, so that it is passed on to successive generations.

In addition to the compositions and methods of the present invention described above, the present invention also provides a method of controlling weeds growing in the:vicinity of the transgenic plants described above. These methods comprise applying a CBI compound to weeds in the vicinity of a transgenic plant having increased resistance to a CBI compound as compared to a wild type variety of the plant. In a preferred embodiment, the transgenic plant comprises a CBIRP coding nucleic acid. In a more preferred embodiment, the transgenic plant comprises an altered PDS nucleic acid. In a more preferred embodiment, the altered PDS nucleic acid encodes an altered PDS protein from *Arabidopsis thaliana* comprising an arginine to proline, amino acid substitution a position corresponding to position 288 in a wild type PDS protein from *Arabidopsis thaliana*. It is envisaged that, the altered PDS nucleic acid encodes an altered PDS protein from *Synechocystis* PCC6803 comprising an alanine to threonine amino acid substitution at a position corresponding to position 175 in a wild type PDS protein from *Synechocystis* PCC6803. The invention provides that the CBIRP coding nucleic acid is an altered PDS polynucleotide of *Arabidopsis thaliana* as defined in SEQ ID NO:1. .

By providing for transgenic plants having increased resistance to a CBI compound, the present invention makes it possible to employ a wide variety of formulations to protect the transgenic plants from weeds, thereby enhancing plant growth and reducing competition for nutrient. A CBI compound can be used by itself for post-emergence control of weeds in areas surrounding the transgenic plants described herein. Alternatively, an herbicide formulation can be used that contains other additives. Such additives include other herbicides, detergents, adjuvants, spreading agent, sticking agents, stabilizing agents, or the like. The CBI compound formulation can be a wet or dry preparation and can include, but is not limited to, flowable powders, emulsifiable concentrates, and liquid concentrates. The CBI compound and compound formulations can be applied in accordance with conventional methods, for example, by spraying, irrigation, dusting, or the like.

The present invention further 33 describes a method for identifying a novel CBIRP, comprising (a) treating one or more organisms with a CBI compound, (b) screening for an organism that has increased resistance to the CBI compound as demonstrated by the organisms surviving the treatment with the CBI compound, and (c) identifying a mutation that contributed to the increased CBI resistance. Alternatively, the present invention describes another method for identifying a novel CBIRP, comprising (a) performing EMS mutagenesis of one or more organisms to introduce random mutations throughout the genome, (b) treating the one or more organisms with a CBI compound, (c) screening for an organism with increased CBI resistance as indicated by the organisms surviving the treatment with the CBI compound, and (d) identifying a mutation that contributed to the increased resistance to the CBI compound.

The invention is further illustrated by the following examples.

### EXAMPLES

### Example 1

### Construction of Vectors Containing an Altered PDS Nucleic Acid from Arabidopsis thaliana

Both nuclear transformation vectors and plastid transformation vectors were constructed comprising mutant *Arabidopsis thaliana* PDS nucleic acids. Plasmids pACBC1000 and pACBC1001 are nuclear transformation vectors comprising a mutant PDS nucleic acid encoding a PDS protein with an arginine to serine amino acid substitution at position 288 of a wild type *A. thaliana* PDS protein. Both plasmids comprise the arginine to serine PDS mutant (R288S) nucleic acid operably linked to the (Ocs)3Mas superpromoter and the Nos terminator. These vectors further comprise a selectable marker nucleic acid operably linked to a plant expressable promoter. The two vectors differ in the orientation of the PDS nucleic acid.

Plasmid p121-I is a plastid transformation vector which comprises the tobacco 16S chloroplast promoter operably linked to a mutant PDS nucleic acid and an *aadA* selectable marker nucleic acid. The mutant PDS nucleic acid in p121-I contains a guanine guanine to cytosine cytosine double base pair substitution, encoding a PDS protein with an arginine to proline amino acid substitution at position 288 of a wild type *A. thaliana* PDS protein.

### Example 2

### Identification of an Altered PDS nucleic acid from Synechocystis PCC6803

Various methods were used to select for spontaneous as well as EMS induced mutants of Synechocystis resistant to AC900001. To isolate spontaneous mutants, wild type Synechocystis was either grown in liquid culture or directly plated on plates containing a lethal concentration of AC900001, or through step-wise exposure to increasing levels of AC900001 in liquid culture. Putative resistant cultures were then placed on selection plates to obtain single resistant cell lines. For isolating EMS induced mutants, Synechocystis cell cultures were treated with EMS at a concentration with 99% lethality and followed by growth on selection plates. A total of 11 independent resistant cell lines were isolated using these procedures.

Genomic DNA was prepared from the EMS induced CBI resistant *Synechocystis* PCC6803 cell lines. PCR was used to amplify a 1.7 kb DNA fragment that encompasses the PDS coding nucleic acid from the genomic DNA. The 1.7 kb fragment from each line was cloned into the TOPO TA cloning vector pCR2.1-TOPO (Qiagen) and sequenced. The PDS gene of one line with increased CBI resistance contained a single base pair substitution of guanine to adenine at position 523 within the open reading frame (ORF), which resulted in a single amino acid substitution of alanine to threonine at position 175 of PDS.

Plastid transformation vectors were constructed comprising a mutant *Synechocystis* PCC6803 PDS nucleic acid, encoding a PDS protein with an alanine to threonine amino acid substitution at position 175 of a wild type *Synechocystis* PCC6803 PDS protein. Plasmids p127-I and p127-II are plastid transformation vectors which comprise the tobacco 16S chloroplast promoter operably linked to the mutant *Synechocystis* PCC6803 PDS nucleic acid and an *aadA* selectable marker nucleic acid. The two vectors differ in the orientation of the PDS nucleic acid.

### Example 3

### Construction of transgenic plants using a nuclear expression vector

Tobacco plants cv Wisconsin were aseptically grown on MS medium containing 3% sucrose and solidified with 0.7% agar. *Agrobacterium tumefaciens* LBA 4404 strains containing either vector pACBC1001 or pACBC1000 were grown in LB medium, and the overnight grown cultures were diluted 1:1 with liquid MS medium. The pACBC1001 and pACBC1000 vectors contain the R288S *A. thaliana* PDS mutant and a selectable marker gene.

Leaves of tobacco plants were cut into pieces (1 cm x 1 cm) and placed in diluted suspension of Agrobacterium for 5 to 10 minutes. Then leaf segments were blotted dry with sterile filter paper and placed on surface of MS media in sterile Petri dishes. The dishes were sealed with Parafilm and incubated at 25°C in the dark. After 2 days of cocultivation, segments of leaves were transferred to regeneration selection medium (MS containing 2 mg/l BAP, 0.1 mg/l NAA, a selection compound, and 500 mg/l cefotaxime). About seven explants were transferred to each 100 mm Petri plate containing 25 ml media. Plates were sealed with Parafilm and cultivated under a 16-hour photoperiod.

Putative transgenic shoots appeared after 4-6 weeks. These shoots were separated from explants and transferred to Magenta boxes containing MS media with a selection compound and 300 mg/l cefotaxime. The Magenta boxes were kept under a 16-hour photoperiod in the culture room. Transgenic plants formed roots on media with selection. PCR analysis confirmed that transgenic lines have been obtained after transformation with pACBC1001 and pACBC1000 constructs.

### Example 4

### Construction of transgenic plants using a plastid expression vector

Leaves were cut and placed abaxial side down on a regeneration medium (Msh-medium supplemented with zeatin (2 mg/L), 1-naphthaleneacetic acid (0.1 mg/L), and sucrose (20 g/L)). Five mg of 0.6 µm gold particles were weighed out and distributed into Treff tubes, followed by washing once with 100 % ethanol and once with sterile bidistilled water. The gold was pelleted and resuspended in 230 µL water and mixed with 20 µg DNA, 250 µL 2.5 M CaCl₂, and 50 µL 0.1 M free base spermidine. The DNA used for plastid transformation was the p121-I plasmid which comprises the mutant *A. thaliana* PDS mutant nucleic acid, encoding a mutant PDS protein with an arginine to proline substitution at position 288 of the wild type *A. thaliana* PDS protein. The gold/DNA mixture was incubated on ice for 10 minutes, centrifuged, and washed twice with 100 % ethanol. After resuspending the gold/DNA mixture in 72 µL of 100 % ethanol, 5.4 mL of the resuspension was applied to each macrocarrier. The macrocarriers were placed in a dessicator for at least one minute. Particle bombardments with the tobacco cultivar Wisconsin-38 were carried out using the DuPont PDS 1000He Biolistic gun with 900 psi rupture discs, a Helium pressure of 1100 psi, and a vacuum level of 27" mercury.

Two days after the bombardment, leaves were cut into 1 cm² pieces and placed on a selective regeneration medium containing spectinomycin (500 mg/L). Regenerants found on different leaf segments were treated as separate transformation events and assigned a different line number. Leaf segments from first round regenerants were placed on the same selection medium. Leaf segments from second round regenerants were placed on two parallel selection plates, one plate containing only spectinomycin (500 mg/L) and the other plate containing both spectinomycin (500 mg/L) and streptomycin (500 mg/L). If leaf segments remained green and showed signs of callus formation (or regeneration) on the dual selection medium, the parallel leaf segments (grown only on spectinomycin (500 mg/L)) were placed on a regeneration medium containing only spectinomycin (500 mg/L). The leaf segments were not taken from the regenerants grown under dual selection because of the potentially mutagenic effects of streptomycin. Fourth round regenerants were transferred to magenta boxes until sufficient roots were exhibited to warrant transplantation to the greenhouse.

Rather than using antibiotic selection, direct selection of leaf segments containing the CBIRP nucleic acid was conducted using the CBI compound AC 375488. Killing curves utilizing AC 375488 were analyzed, and the most efficacious selection rate was determined to be 0.07 µM. Following bombardment, leaf segments were transferred to a regeneration medium containing 0.07 µM AC 375488 and 10 g/L sucrose (as opposed to the usual 20 g/L). Three weeks following the bombardment, leaf explants were transferred to a regeneration medium with 0.1 µM AC 375488 and 5 g/L sucrose. After three more weeks, leaf explants were transferred to the same selection medium for regeneration.

### Example 5

### Assessment of CBI resistance of the transgenic plants

Several regeneration assays were performed on wild type and putative transgenic lines in the presence of fluridone. In the first assay, petri plates were made with Msh-, 2 mg/L zeatin, 0.1 mg/L NAA, and a range of fluridone concentrations (0, 0.025, 0.05, 0.1, and 0.2 µM fluridone). The first round of explants were regenerated on a medium containing spectinomycin (500 mg/L), and the second round of regenerants were obtained on a medium containing 3.0 µM fluridone. Explants from one line, G-990630-1.2 (containing an altered *A. thaliana* PDS nucleic acid encoding an arginine to proline amino acid substitution as represented by SEQ ID NO:2), regenerated and remained green at each of the fluridone concentrations. Wild type explants regenerated at all fluridone concentrations, but the tissue was bleached white.

In the second assay, leaf and stem segments from a putative transgenic line, G-990623-8.1 (containing an altered *A. thaliana* PDS nucleic acid encoding an arginine to proline amino acid substitution as represented by SEQ ID NO:2) were transferred to different selection plates. Petri plates were made with a regeneration medium (Msh-, 2 mg/L zeatin, 0.1 mg/L NAA), supplemented with 0.05 µM fluridone, 0.1 µM fluridone, or 500 mg/L spectinomycin with 500 mg/L streptomycin. Rooting/shooting plates (Msh-, 1 mg/L BAP) supplemented with 0.5 µM fluridone were also made. All leaf explants from this line that were transferred to the regeneration medium containing fluridone displayed numerous shoots. Leaf segments from this line that were transferred to a regeneration medium containing spectinomycin and streptomycin remained green and exhibited tissue expansion and callus-like growth. In addition, stem segments from this line that were transferred to the rooting/shooting medium displayed vigorous rooting and shooting.

A similar *in vitro* shoot induction assay was performed for nuclear transgenic lines on media with different concentrations of fluridone or norflurazon. The nuclear transgenic lines were transformed with either the pACBC1001 or pACBC1000 vector (*A. thaliana* PDS mutant R288S). Segments of leaves of control lines and transgenic lines were placed on MS regeneration media with different fluridone concentrations (0 µM, 0.25 µM, 1 µM, or 3 pM). Control non-transgenic explants produced only bleaching shoots at all fluridone concentrations. Transgenic lines produced green shoots at 0.25 µM and 1 µM fluridone, and two of these transgenic lines also produced green shoots at 3 µM fluridone. The norflurazon resistance of the nuclear transgenic lines also has been tested. Segments of leaves were placed on MS regeneration media with different norflurazon concentrations (0 µM, 0.5 µM, 1 µM, or 2.5 µM). Control non-transgenic explants produced only bleaching shoots at 1 µM and 2.5 µM norflurazon concentrations. Eight transgenic lines tested produced green shoots at 1 µM, or 2.5 µM norflurazon concentrations.

In addition, several spray tests were performed on wild type and putative transgenic or transplastomic lines. The tobacco plants were typically grown to about 4 cm tall, about 10 cm in diameter, and to the four leaf stage before sprayed. In the first test, control non-transgenic plants and transplastomic lines comprising the p121-I vector *(A. thaliana* PDS mutant R288P) were sprayed with varying concentrations of norflurazon (0, 10, 20, and 40 g/ha). The control non-transgenic plants showed bleaching symptoms beginning at 10 g/ha norflurazon, and the transplastomic lines did not show bleaching at any of these concentrations In the second spray test, control non-transgenic plants and transplastomic lines comprising the p121-I vector *(A. thaliana* PDS mutant R288P) were sprayed with varying concentrations of norflurazon (0, 80, 160, 320, and 640 g/ha). Again, the transplastomic lines did not show any signs of damage at any norflurazon concentration.

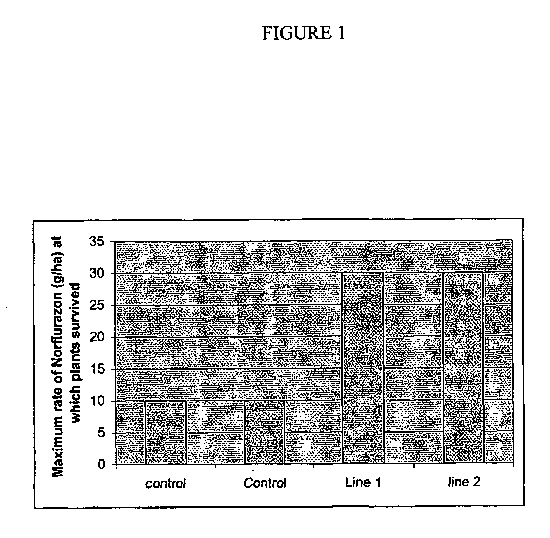

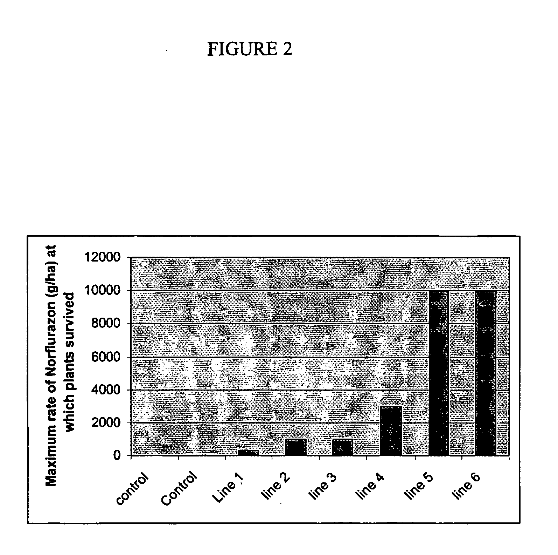

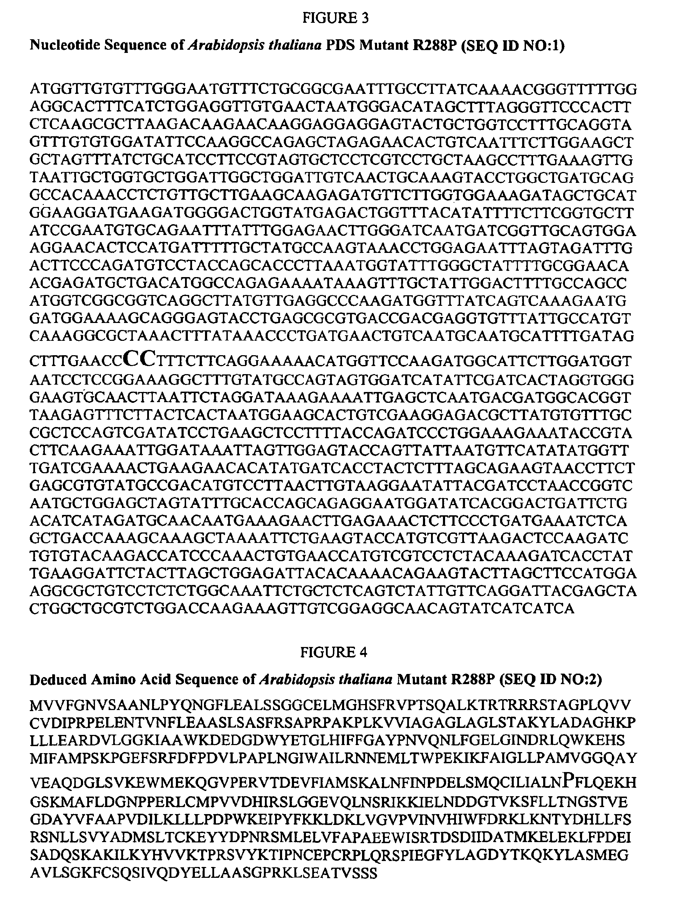

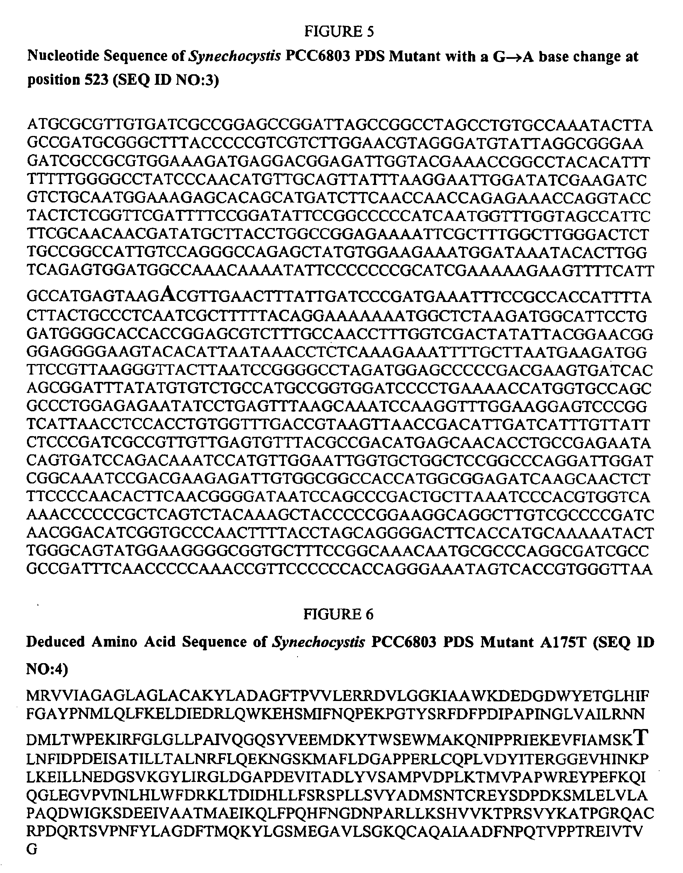

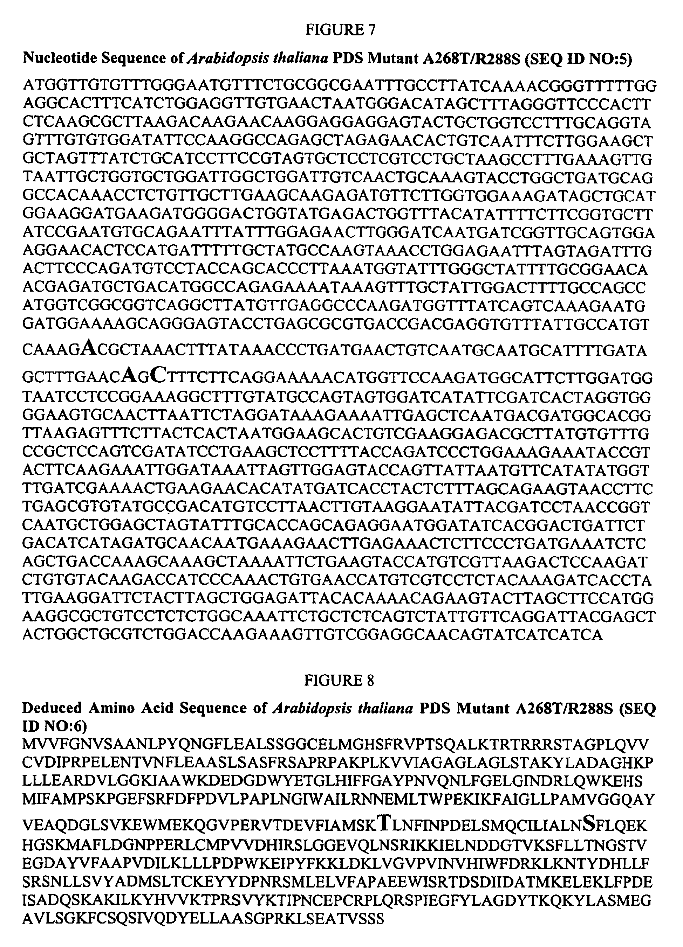

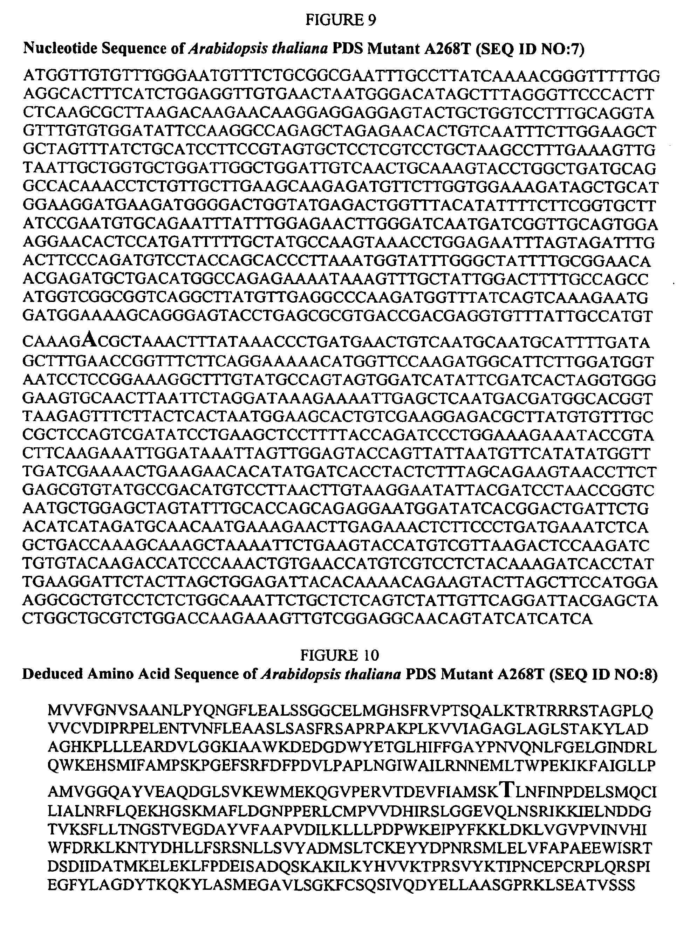

In a third spray test, control non-transgenic plants, nuclear transgenic lines (comprising the pACBC1001 or pACBC1000 vector (*A. thaliana* PDS mutant R288S)), and transplastomic lines (comprising the p121-I vector (*A. thaliana* PDS mutant R288P)) were sprayed with varying concentrations of norflurazon (0, 30, 100, 300, 1,000, and 3,000 g/ha), fluridone (10, 30, and 100 g/ha), or AC900001 (10, 30, and 100 g/ha). The control non-transgenic plants showed bleaching at all concentrations of each of the herbicides. At all concentrations of fluridone and AC900001, the nuclear transgenic lines and transplastomic lines demonstrated bleaching similar to the non-transgenic plants. The nuclear transgenic lines showed a lower level of bleaching as compared to the control plants at 10 g/ha norflurazon; however, at higher concentrations of norflurazon, the nuclear transgenic lines demonstrated bleaching similar to the non-transgenic plants (Figure 1). By contrast, transplastomic lines comprising the p121-I vector did not show bleaching even at the highest concentrations (1,000 g/ha and 3,000 g/ha), demonstrating a 100-fold to 300-fold increase in CBI resistance. Some of the transplastomic lines showed minimal leaf necrosis at norflurazon concentrations of 300, 1,000, and 3,000 g/ha In a fourth spray test, two transplastomic lines comprising the p121-I vector did not show bleaching injury at 10,000 g/ha (Figure 2). In the fourth spray test, transplastomic lines comprising the p122-I or p122-II vector (*A. thaliana* PDS mutant R288S) showed significant bleaching and scorching injury at all concentrations of norflurazon (0, 123, 370, 1,111, 3,333, and 10,000 g/ha), fluridone (11, 33, and 100 g/ha), and AC 900001 (11, 33, and 100 g/ha).

### Example 6

### Engineering herbicide-tolerant soybean plants by overexpressing a CBIRP nucleic acid

The CBIRP vectors described above are used to transform soybean as described below.

Seeds of soybean are surface sterilized with 70% ethanol for 4 minutes at room temperature with continuous shaking, followed by 20% (v/v) Clorox supplemented with 0.05% (v/v) Tween for 20 minutes with continuous shaking. Then, the seeds are rinsed 4 times with distilled water and placed on moistened sterile filter paper in a Petri dish at room temperature for 6 to 39 hours. The seed coats are peeled off, and cotyledons are detached from the embryo axis. The embryo axis is examined to make sure that the meristematic region is not damaged. The excised embryo axes are collected in a half-open sterile Petri dish and air-dried to a moisture content less than 20% (fresh weight) in a sealed Petri dish until further use.

*Agrobacterium tumefaciens* culture is prepared from a single colony in LB solid medium plus appropriate antibiotics (e.g. 100 mg/l streptomycin) followed by growth of the single colony in liquid LB medium to an optical density at 600 nm of 0.8. Then, the bacteria culture is pelleted at 7000 rpm for 7 minutes at room temperature, and resuspended in MS (Murashige and Skoog, 1962) medium supplemented with 100 µM acetosyringone. Bacteria cultures are incubated in this pre-induction medium for 2 hours at room temperature before use. The axis of soybean zygotic seed embryos at approximately 15% moisture content are imbibed for 2 hours at room temperature with the pre-induced *Agrobacterium* suspension culture. The embryos are removed from the imbibition culture and transferred to Petri dishes containing solid MS medium supplemented with 2% sucrose and incubated for 2 days, in the dark at room temperature. Alternatively, the embryos are placed on top of moistened (liquid MS medium) sterile filter paper in a Petri dish and incubated under the same conditions described above. After this period, the embryos are transferred to either solid or liquid MS medium supplemented with 500 mg/L carbenicillin or 300mg/L cefotaxime to kill the agrobacteria. The liquid medium is used to moisten the sterile filter paper. The embryos are incubated during 4 weeks at 25°C, under 150 µmol m⁻²sec⁻¹ and 12 hours photoperiod. Once the seedlings produced roots, they are transferred to sterile metromix soil. The medium of the *in vitro* plants is washed off before transferring the plants to soil. The plants are kept under a plastic cover for 1 week to favor the acclimatization process. Then the plants are transferred to a growth room where they are incubated at 25°C, under 150 µmol m⁻²sec⁻¹ light intensity and 12 hours photoperiod for about 80 days.

The transgenic plants are then screened for their increased CBI resistance according to the methods described in Example 5 demonstrating that transgene expression increases CBI resistance.

### Example 7

### Engineering herbicide-tolerant Rapeseed/Canola plants by overexpressing a CBIRP nucleic acid

The CBIRP vectors described above are used to transform rapeseed/canola as described below.

The method of plant transformation described herein is also applicable to *Brassica* and other crops. Seeds of canola are surface sterilized with 70% ethanol for 4 minutes at room temperature with continuous shaking, followed by 20% (v/v) Clorox supplemented with 0.05 % (v/v) Tween for 20 minutes, at room temperature with continuous shaking. Then, the seeds are rinsed 4 times with distilled water and placed on moistened sterile filter paper in a Petri dish at room temperature for 18 hours. Then the seed coats are removed and the seeds are air dried overnight in a half-open sterile Petri dish. During this period, the seeds lose approx. 85% of its water content. The seeds are then stored at room temperature in a sealed Petri dish until further use. DNA constructs and embryo imbibition are as described in Example 7. Samples of the primary transgenic plants (T0) are analyzed by PCR to confirm the presence of T-DNA. These results are confirmed by Southern hybridization in which DNA is electrophoresed on a 1% agarose gel and transferred to a positively charged nylon membrane (Roche Diagnostics). The PCR DIG Probe Synthesis Kit (Roche Diagnostics) is used to prepare a digoxigenin-labelled probe by PCR, and used as recommended by the manufacturer.

The transgenic plants are then screened for their increased CBI resistance according to the methods described in Example 5 demonstrating that transgene expression increases CBI resistance.

### Example 8

### Engineering herbicide-tolerant corn plants by overexpressing a CBIRP nucleic acid

The CBIRP vectors described above are used to transform corn as described below.

Transformation of maize (*Zea Mays L.*) is performed with the method described by Ishida et al. 1996. Nature Biotech 14:745-50. Immature embryos are co-cultivated with *Agrobacterium tumefaciens* that carry "super binary" vectors, and transgenic plants are recovered through organogenesis. This procedure provides a transformation efficiency of between 2.5% and 20%. The transgenic plants are then screened for their increased CBI resistance according to the methods described in Example 5 demonstrating that transgene expression increases CBI resistance.

### Example 9

### Engineering herbicide-tolerant wheat plants by over-expressing a CBIRP nucleic acid

The CBIRP vectors described above are used to transform wheat as described below or as described in the literature.

Transformation of wheat is performed with the method described by Ishida et al. 1996 Nature Biotech. 14:745-50. Immature embryos are co-cultivated with *Agrobacterium tumefaciens* that carry "super binary" vectors, and transgenic plants are recovered through organogenesis. This procedure provides a transformation efficiency between 2.5% and 20%. The transgenic plants are then screened for their increased CBI resistance according to the methods described in Example 5 demonstrating that transgene expression increases CBI resistance.

### Example 10

### Engineering herbicide-tolerant barley plants by over-expressing a CBIRP nucleic acid

The CBIRP vectors described above are used to transform barley as described below or as described in the literature.

Transformation of wheat is performed with the method described by Ishida et al. 1996 Nature Biotech. 14:745-50. Immature embryos are co-cultivated with *Agrobacterium tumefaciens* that carry "super binary" vectors, and transgenic plants are recovered through organogenesis. This procedure provides a transformation efficiency between 2.5% and 20%. The transgenic plants are then screened for their increased CBI resistance according to the methods described in Example 5 demonstrating that transgene expression increases CBI resistance.

## Claims

1. A transgenic plant cell comprising a carotenoid biosynthesis inhibitor resistant protein (CBIRP) coding nucleic acid, wherein the CBIRP coding nucleic acid comprises a polynucleotide sequence selected from the group consisting of:
a) a polynucleotide of SEQ ID NO:1;
b) a polynucleotide encoding a polypeptide of SEQ ID NO:2; and
c) a polynucleotide encoding a biologically active portion of the polypeptide of SEQ ID NO: 2 of at least 100 amino acids in length, wherein said biologically active portion of the polypeptide comprises position 288 of SEQ ID NO:2.

2. The transgenic plant cell of Claim 1, wherein the CBIRP coding nucleic acid is a polynucleotide as defined in SEQ ID NO:1, and wherein
a) expression of the nucleic acid in the plant cell results in the plant cell's increased resistance to a CBI compound as compared to a wild type variety of the plant cell, and
b) the CBIRP is a mutant phytoene desaturase (PDS) polypeptide from *Arabidopsis thaliana.*

3. The transgenic plant cell of Claim 1, wherein the CBI compound is selected from the group consisting of fluridone, norflurazon, AC 374588, and AC 900001.

4. The transgenic plant cell of Claim 1, wherein the plant is a monocot.

5. The transgenic plant cell of Claim 1, wherein the plant is a dicot.

6. The transgenic plant cell of Claim 1, wherein the plant is selected from the group consisting of maize, wheat, rye, oat, triticale, rice, barley, soybean, peanut, cotton, radeseed, canola, manihot, pepper, sunflower, tagetes, solanaceous plants, potato, tobacco, eggplant, tomato, Vicia species, pea, alfalfa, coffee, cacao, tea, Salix species, oil palm, coconut, perennial grass, and a forage crop.

7. The transgenic plant cell of Claim 1, wherein the plant is selected from the group consisting of barley, wheat, canola, corn, and soybean.

8. A transgenic plant comprising a plant cell according to any one of Claims 1 to 7.

9. A seed produced by a transgenic plant comprising a plant cell according to any of Claims 1 to 7, wherein the seed comprises the nucleic acid of SEQ ID NO: 1 and is true breeding for an increased resistance to a CBI compound as compared to a wild type variety of the plant cell.

10. An isolated carotenoid biosynthesis inhibitor resistant protein (CBIRP) coding nucleic acid, wherein the nucleic acid comprises a polynucleotide sequence selected from the group consisting of:
a) a polynucleotide of SEQ ID NO: 1;
b) a polynucleotide encoding a polypeptide of SEQ ID NO:2; and
c) a polynucleotide encoding a biologically active portion of the polypeptide of SEQ ID NO: 2 of at least 100 amino acids in length, wherein said biologically active portion of the polypeptide comprises position 288 of SEQ ID NO. 2.

11. A method for producing a transgenic plant, comprising
a) transforming a plant cell with a carotenoid biosynthesis inhibitor resistant protein (CBIRP) coding nucleic acid and
b) generating from the plant cell the transgenic plant, wherein the transgenic plant has increased resistance to a CBI compound as compared to a wild type variety of the plant,
wherein the nucleic acid comprises a polynucleotide sequence selected from the group consisting of:
a) a polynucleotide of SEQ ID NO:1;
b) a polynucleotide encoding a polypeptide of SEQ ID NO:2; and
c) a polynucleotide encoding a biologically active portion of the polypeptide of SEQ ID NO: 2 of at least 100 amino acids in length, wherein said biologically active portion of the polypeptide comprises position 288 of SEQ ID. NO. 2.

12. The method of Claim 11, wherein the CBIRP coding nucleic acid comprises a polynucleotide selected from the group consisting of:
a) a polynucleotide of SEQ ID NO:1;
b) a polynucleotide encoding a polypeptide of SEQ ID NO:2; and
c) a polynucleotide encoding a biologically active portion of the polypeptide of SEQ ID NO: 2 of at least 100 amino acids in length, wherein said biologically active portion of the polypeptide comprises position 288 of SEQ ID NO: 2.

13. A method of controlling weeds within the vicinity of a transgenic plant, comprising applying a CBI compound to the weeds and to the transgenic plant, wherein; the transgenic plant comprises a polynucleotide encoding a carotenoid biosynthesis, inhibitor resistant protein (CBIRP) comprising an altered PDS protein from *Aradidopsis thaliana* comprising position 288 of SEQ ID NO:2.

14. The method of Claim 13, wherein the transgenic plant comprises a polynucleotide sequence selected from the group consisting of:
a) a polynucleotide of SEQ ID NO:1;
b) a polynucleotide encoding a polypeptide of SEQ ID NO:2; and
c) a polynucleotide encoding a biologically active portion of the polypeptide of SEQ ID NO: 2 of at least 100 amino acids in length, wherein said biologically active portion of the polypeptide comprises position 288 of SEQ ID NO: 2.

15. The method of Claim 13, wherein the CBI compound is selected from the group consisting of fluridone, norflurazon, AC 375488, and AC 900001.

## Patentansprüche

1. Transgene Pflanzenzelle, die eine Nukleinsäure, die für ein Protein für Resistenz gegen einen Carotinoidbiosynthesehemmer (CBIRP) codiert, umfaßt, wobei die für das CBIRP codierende Nukleinsäure eine Polynukleotidsequenz aus der folgenden Gruppe umfaßt:
a) ein Polynukleotid gemäß SEQ ID NO:1;
b) ein Polynukleotid, das für ein Polypeptid gemäß SEQ ID NO:2 codiert; und
c) ein Polynukleotid, das für einen biologisch aktiven Abschnitt des Polypeptids gemäß SEQ ID NO:2 mit einer Länge von mindestens 100 Aminosäuren codiert, wobei der biologisch aktive Abschnitt des Polypeptids Position 288 von SEQ ID NO:2 umfaßt.

2. Transgene Pflanzenzelle nach Anspruch 1, wobei es sich bei der für das CBIRP codierenden Nukleinsäure um ein Polynukleotid wie in SEQ IN NO:1 definiert handelt, und wobei
a) die Expression der Nukleinsäure in der Pflanzenzelle dazu führt, daß die Pflanzenzelle im Vergleich zu einer Wildtypart dieser Pflanzenzelle eine erhöhte Resistenz gegen eine CBI-Vebindung aufweist, und
b) es sich bei dem CBIRP um ein mutiertes Phytoendesaturase (PDS)-Polypeptid aus *Arabidopsis thaliana* handelt.

3. Transgene Pflanzenzelle nach Anspruch 1, wobei die CBI-Verbindung aus der Gruppe Fluridon, Norflurazon, AC 374588 und AC 900001 stammt.

4. Transgene Pflanzenzelle nach Anspruch 1, wobei es sich bei der Pflanze um eine einkeimblättrige Pflanze handelt.

5. Transgene Pflanzenzelle nach Anspruch 1, wobei es sich bei der Pflanze um eine zweikeimblättrige Pflanze handelt.

6. Transgene Pflanzenzelle nach Anspruch 1, wobei die Pflanze aus der Gruppe Mais, Weizen, Roggen, Hafer, Triticale, Reis, Gerste, Sojabohne, Erdnuß, Baumwolle, Raps, Canola, Maniok, Pfeffer, Sonnenblume, Tagetes, Solanaceen-Pflanzen, Kartoffel, Tabak, Aubergine, Tomate, Vicia-Arten, Erbse, Luzerne, Kaffee, Kakao, Tee, Salix-Arten, Ölpalme, Kokosnuß, mehrjährige Gräser und Futterkultur stammt.

7. Transgene Pflanzenzelle nach Anspruch 1, wobei die Pflanze aus der Reihe Gerste, Weizen, Canola, Mais und Sojabohne stammt.

8. Transgene Pflanze, die eine Pflanzenzelle nach einem der Ansprüche 1 bis 7 umfaßt.

9. Samen, der von einer transgenen Pflanze, die eine Pflanzenzelle nach einem der Ansprüche 1 bis 7 umfaßt, produziert wird, wobei der Samen die Nukleinsäure gemäß SEQ ID NO:1 umfaßt und für eine im Vergleich zu einer Wildtypart der Pflanzenzelle erhöhte Resistenz gegen eine CBI-Verbindung reinerbig ist.

10. Isolierte Nukleinsäure, die für ein Protein für Resistenz gegen einen Carotinoidbiosynthesehemmer (CBIRP) codiert, wobei die Nukleinsäure eine Polynukleotidsequenz aus der folgenden Gruppe umfaßt:
a) ein Polynukleotid gemäß SEQ ID NO:1;
b) ein Polynukleotid, das für ein Polypeptid gemäß SEQ ID NO:2 codiert; und
c) ein Polynukleotid, das für einen biologisch aktiven Abschnitt des Polypeptids gemäß SEQ ID NO:2 mit einer Länge von mindestens 100 Aminosäuren codiert, wobei der biologisch aktive Abschnitt des Polypeptids Position 288 von SEQ ID NO:2 umfaßt.

11. Verfahren zur Herstellung einer transgenen Pflanze, das folgendes umfaßt:
a) Transformieren einer Pflanzenzelle mit einer für ein Protein für Resistenz gegen einen Carotinoidbiosynthesehemmer (CBIRP) codierenden Nukleinsäure; und
b) Erzeugen der transgenen Pflanze aus der Pflanzenzelle, wobei die transgene Pflanze eine im Vergleich zu einer Wildtypart der Pflanze erhöhte Resistenz gegen eine CBI-Verbindung aufweist,
wobei die Nukleinsäure eine Polynukleotidsequenz aus der folgenden Gruppe umfaßt:
a) ein Polynukleotid gemäß SEQ ID NO:1;
b) ein Polynukleotid, das für ein Polypeptid gemäß SEQ ID NO:2 codiert; und
c) ein Polynukleotid, das für einen biologisch aktiven Abschnitt des Polypeptids gemäß SEQ ID NO:2 mit einer Länge von mindestens 100 Aminosäuren codiert, wobei der biologisch aktive Abschnitt des Polypeptids Position 288 von SEQ ID NO:2 umfaßt.

12. Verfahren nach Anspruch 11, wobei die für das CBIRP codierende Nukleinsäure eine Polynukleotidsequenz aus der Gruppe
a) ein Polynukleotid gemäß SEQ ID NO:1;
b) ein Polynukleotid, das für ein Polypeptid gemäß SEQ ID NO:2 codiert; und
c) ein Polynukleotid, das für einen biologisch aktiven Abschnitt des Polypeptids gemäß SEQ ID NO:2 mit einer Länge von mindestens 100 Aminosäuren codiert, wobei der biologisch aktive Abschnitt des Polypeptids Position 288 von SEQ ID NO:2 umfaßt.
umfaßt.

13. Verfahren zur Bekämpfung von Unkräutern in der Nähe einer transgenen Pflanze, bei dem man eine CBI-Verbindung auf die Unkräuter und auf die transgene Pflanze ausbringt, wobei die transgene Pflanze ein für ein Protein für Resistenz gegen einen Carotinoidbiosynthesehemmer (CBIRP) codierendes Polynukleotid umfassend ein verändertes PDS-Protein aus *Arabidopsis thaliana* umfassend Position 288 gemäß SEQ ID NO:2 codiert, umfaßt.

14. Verfahren nach Anspruch 13, wobei die transgene Pflanze eine Polynukleotidsequenz aus der folgenden Gruppe umfaßt:
a) ein Polynukleotid gemäß SEQ ID NO:1;
b) ein Polynukleotid, das für ein Polypeptid gemäß SEQ ID NO:2 codiert; und
c) ein Polynukleotid, das für einen biologisch aktiven Abschnitt des Polypeptids gemäß SEQ ID NO:2 mit einer Länge von mindestens 100 Aminosäuren codiert, wobei der biologisch aktive Abschnitt des Polypeptids Postition 288 von SEQ ID NO:2 umfaßt.

15. Verfahren nach Anspruch 13, wobei die CBI-Verbindung aus der Gruppe Fluridon, Norflurazon, AC 375488 und AC 900001 stammt.

## Revendications

1. Cellule végétale transgénique comprenant un acide nucléique codant pour une protéine résistant aux inhibiteurs de la biosynthèse des caroténoïdes (CBIRP), **caractérisée en ce que** l'acide nucléique codant pour la CBIRP comprend une séquence polynucléotidique choisie dans le groupe constitué :
a) d'un polynucleotide de SEQ ID NO : 1 ;
b) d'un polynucleotide codant pour un polypeptide de SEQ ID NO : 2 ; et
c) d'un polynucleotide codant pour une partie biologiquement active du polypeptide de SEQ ID NO : 2 d'au moins 100 acides aminés de long, ladite partie biologiquement active du polypeptide comprenant la position 288 de SEQ ID NO : 2.

2. Cellule végétale transgénique selon la revendication 1, **caractérisée en ce que** l'acide nucléique codant pour la CBIRP est un polynucléotide tel que défini dans SEQ ID NO : 1 et **en ce que**
a) l'expression de l'acide nucléique dans la cellule végétale conduit à une résistance accrue de la cellule végétale à un composé CBI par rapport à une variété de type sauvage de la cellule végétale, et
b) la CBIRP est un polypeptide de phytoène désaturase (PDS) mutant d'*Arabidopsis thaliana.*

3. Cellule végétale transgénique selon la revendication 1, **caractérisée en ce que** le composé CBI est choisi dans le groupe constitué de la fluridone, du norflurazon, de l'AC 374588 et de l'AC 900001.

4. Cellule végétale transgénique selon la revendication 1, **caractérisée en ce que** la plante est une monocotylédone.

5. Cellule végétale transgénique selon la revendication 1, **caractérisée en ce que** la plante est une dicotylédone.

6. Cellule végétale transgénique selon la revendication 1, **caractérisée en ce que** la plante est choisie dans le groupe constitué du maïs, du blé, du seigle, de l'avoine, du triticale, du riz, de l'orge, du soja, de l'arachide, du coton, du colza, du canola, du manioc, du poivron, du tournesol, du tagète, des Solanacées, de la pomme de terre, du tabac, de l'aubergine, de la tomate, des espèces de Vicia, du pois, de la luzerne, du café, du cacao, du thé, des espèces de Salix, du palmier à huile, de la noix de coco, d'une herbe vivace et d'une culture fourragère.

7. Cellule végétale transgénique selon la revendication 1, **caractérisée en ce que** la plante est choisie dans le groupe constitué de l'orge, du blé, du canola, du maïs et du soja.

8. Plante transgénique comprenant une cellule végétale selon l'une quelconque des revendications 1 à 7.

9. Graine produite par une plante transgénique comprenant une cellule végétale selon l'une quelconque des revendications 1 à 7, **caractérisée en ce que** la graine comprend l'acide nucléique de SEQ ID NO : 1 et est de lignée pure pour ce qui est d'une résistance accrue à un composé CBI par rapport à une variété de type sauvage de la cellule végétale.

10. Acide nucléique isolé codant pour une protéine résistant aux inhibiteurs de la biosynthèse des caroténoïdes (CBIRP), **caractérisé en ce que** l'acide nucléique comprend une séquence polynucléotidique choisie dans le groupe constitué :
a) d'un polynucléotide de SEQ ID NO: 1 ;
b) d'un polynucléotide codant pour un polypeptide de SEQ ID NO : 2 ; et
c) d'un polynucléotide codant pour une partie biologiquement active du polypeptide de SEQ ID NO : 2 d'au moins 100 acides aminés de long, ladite partie biologiquement active du polypeptide comprenant la position 288 de SEQ ID NO : 2.

11. Procédé de production d'une plante transgénique comprenant les étapes consistant à :
a) transformer une cellule végétale avec un acide nucléique codant pour une protéine résistant aux inhibiteurs de la biosynthèse des caroténoïdes (CBIRP) ; et
b) générer la plante transgénique à partir de la cellule végétale, la plante transgénique ayant une résistance accrue à un composé CBI par rapport à une variété de type sauvage de la plante,
**caractérisé en ce que** l'acide nucléique comprend une séquence polynucléotidique choisie dans le groupe constitué :
a) d'un polynucleotide de SEQ ID NO : 1 ;
b) d'un polynucléotide codant pour un polypeptide de SEQ ID NO : 2 ; et
c) d'un polynucléotide codant pour une partie biologiquement active du polypeptide de SEQ ID NO : 2 d'au moins 100 acides aminés de long, ladite partie biologiquement active du polypeptide comprenant la position 288 de SEQ ID NO : 2.

12. Procédé selon la revendication 11, **caractérisé en ce que** l'acide nucléique codant pour la CBIRP comprend un polynucléotide choisi dans le groupe constitué :
a) d'un polynucléotide de SEQ ID NO : 1 ;
b) d'un polynucléotide codant pour un polypeptide de SEQ ID NO . 2 ; et
c) d'un polynucléotide codant pour une partie biologiquement active du polypeptide de SEQ ID NO : 2 d'au moins 100 acides aminés de long, ladite partie biologiquement active du polypeptide comprenant la position 288 de SEQ ID NO : 2.

13. Procédé de contrôle des mauvaises herbes aux alentours d'une plante transgénique, comprenant l'étape consistant à appliquer un composé CBI sur les mauvaises herbes et sur la plante transgénique, **caractérisé en ce que** la plante transgénique comprend un polynucléotide codant pour une protéine résistant aux inhibiteurs de la biosynthèse des caroténoïdes (CBIRP) comprenant une protéine PDS altérée d'*Arabidopsis thaliana* comprenant la position 288 de SEQ ID NO : 2.

14. Procédé selon la revendication 13, **caractérisé en ce que** la plante transgénique comprend une séquence polynucléotidique choisie dans le groupe constitué :
a) d'un polynucléotide de SEQ ID NO : 1 ;
b) d'un polynucléotide codant pour un polypeptide de SEQ ID NO : 2 ; et
c) d'un polynucléotide codant pour une partie biologiquement active du polypeptide de SEQ ID NO : 2 d'au moins 100 acides aminés de long, ladite partie biologiquement active du polypeptide comprenant la position 288 de SEQ ID NO : 2.

15. Procédé selon la revendication 13, **caractérisé en ce que** le composé CBI est choisi dans le groupe constitué de la fluridone, du norflurazon, de l'AC 375488 et de l'AC 900001.
